(19) Europäisches Patentamt
European Patent Office
Office européen des brevets

(11) EP 1 883 390 B1

(12) EUROPEAN PATENT SPECIFICATION

(45) Date of publication and mention of the grant of the patent:
**29.07.2009 Bulletin 2009/31**

(21) Application number: **06726913.4**

(22) Date of filing: **26.04.2006**

(51) Int Cl.:
***A61K 8/00** (2006.01)*
***A61K 9/00** (2006.01)*
***A61K 9/06** (2006.01)*
***A61K 31/60** (2006.01)*
***A61K 47/30** (2006.01)*

(86) International application number:
**PCT/GB2006/001527**

(87) International publication number:
**WO 2006/117516 (09.11.2006 Gazette 2006/45)**

(54) **TOPICAL COMPOSITIONS**

TOPISCHE ZUSAMMENSETZUNGEN

COMPOSITIONS TOPIQUES

(84) Designated Contracting States:
**AT BE BG CH CY CZ DE DK EE ES FI FR GB GR HU IE IS IT LI LT LU LV MC NL PL PT RO SE SI SK TR**

(30) Priority: **05.05.2005 GB 0509123**
**12.08.2005 GB 0516558**

(43) Date of publication of application:
**06.02.2008 Bulletin 2008/06**

(73) Proprietor: **Reckitt Benckiser Inc.**
**Parsippany, New Jersey 07054 (US)**

(72) Inventors:
- **KOSTURKO, Richard J.**
**Montvale, NJ 07645 (US)**
- **TAYLOR, Candice, Lida**
**Montvale, NJ 07645 (US)**

(74) Representative: **Bowers, Craig Malcolm et al**
**Reckitt Benckiser**
**Corporate Services Limited**
**Legal Department - Patents Group**
**Dansom Lane**
**Hull**
**HU8 7DS (GB)**

(56) References cited:
**WO-A-87/02891**
**US-A1- 2004 063 794**
**US-A1- 2005 249 689**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 883 390 B1

## Description

The present invention relates to topical compositions for application to human skin. More particularly, the compositions according to the invention are directed to topical compositions which are intended to provide an antimicrobial benefit.

Topical compositions, per se, are well-known in the cosmetic, dermatological as well as in the pharmaceutical fields. Most topical compositions are intended to provide at least one but generally two or more specific benefits after being applied to the human skin. For example, sunscreen compositions are intended to provide a primary benefits in blocking the harmful effects of solar radiation but frequently also providing ancillary benefits such as moisturizing and nourishing the skin. As a further example, nor pharmaceutical topical preparations which are intended to provide a specific therapeutic benefits to skin elements such as acne, eczema, or other skin related afflictions. Yet further topical compositions are primarily intended to be soaps for general cleaning of the human skin such as hand soaps or body wash soaps or care shampoos and other hair treatment compositions.

The compositions of the present invention provide as a primary technical benefit the reduction of undesired microorganisms, particularly in the reduction of both gram positive and gram negative microorganisms, while at the same time in providing secondary benefits including the skin conditioning and/or skin cleansing. Further ancillary benefits may be provided by the presence of one or more for the optional constituents which may be included in formulations or compositions according to the present intervention. These are discussed in more detail hereinafter, particularly are discussed in reference to one or more of the examples set forth below.

Accordingly in one aspect of the invention there is provided an improved topical composition for the application to the human body particularly to the skin or hair and most preferably to the skin which composition provides a useful antimicrobial benefit.

According to a further aspect of the invention there is provided a topical composition which has a useful antimicrobial and a useful skin cleaning/treatment benefit when applied to the skin.

According to a still further aspect of the invention there is provided a topical composition as described previously which is effective in providing an effective antimicrobial benefit against both gram positive and gram negative microorganisms which may present on body surfaces particularly on the skin.

According to a yet further aspect of the invention is provided a method for the manufacture or production on improved topical composition as set forth herein. According to a still further aspect of the inventions is provided an improved method for the treatment of the skin as well as other body surface is including the hair which method contemplates the application of any cleaning and or antimicrobial leak effective amount of the topical composition described herein in order to provide an effective cleaning and/or antimicrobial benefit.

Other features and advantages of the present invention will be apparent from the following detailed description of the invention and from the accompanying claims.

The compositions of the invention include one or more anionic surfactants as well as salt forms thereof. Examples of anionic surfactants include alcohol sulfates and sulfonates, alcohol phosphates and phosphonates, alkyl ester sulfates, alkyl diphenyl ether sulfonates, alkyl sulfates, alkyl ether sulfates, sulfate esters of an alkylphenoxy polyoxyethylene ethanol, alkyl monoglyceride sulfates, alkyl sulfonates, alkyl ether sulfates, alpha-olefin sulfonates, beta-alkoxy alkane sulfonates, alkyl ether sulfonates, ethoxylated alkyl sulfonates, alkylaryl sulfonates, alkylaryl sulfates, alkyl monoglyceride sulfonates, alkyl carboxylates, alkyl ether carboxylates, alkyl alkoxy carboxylates having 1 to 5 moles of ethylene oxide, alkylpolyglycolethersulfates (containing up to 10 moles of ethylene oxide), sulfosuccinates, octoxynol or nonoxynol phosphates, taurates, fatty taurides, fatty acid amide polyoxyethylene sulfates, acyl glycerol sulfonates, fatty oleyl glycerol sulfates, alkyl phenol ethylene oxide ether sulfates, paraffin sulfonates, alkyl phosphates, isethionates, N-acyl taurates, alkyl succinamates and sulfosuccinates, alkylpolysaccharide sulfates, alkylpolyglucoside sulfates, alkyl polyethoxy carboxylates, and sarcosinates or mixtures thereof.

Further examples of anionic surfactants include water soluble salts or acids of the formula $(ROSO_3)_xM$ or $(RSO_3)_xM$ wherein R is preferably a $C_6$-$C_{24}$ hydrocarbyl, preferably an alkyl or hydroxyalkyl having a $C_{10}$-$C_{20}$ alkyl component, more preferably a $C_{12}$-$C_{18}$ alkyl or hydroxyalkyl, and M is H or a mono-, di- or tri-valent cation, e. g., an alkali metal cation (e. g., sodium, potassium, lithium), or ammonium or substituted ammonium (e. g., methyl-, dimethyl-, and trimethyl ammonium cations and quaternary ammonium cations, such as tetramethyl-ammonium and dimethyl piperdinium cations and quaternary ammonium cations derived from alkylamines such as ethylamine, diethylamine, triethylamine, and mixtures thereof, and the like) and x is an integer, preferably 1 to 3, most preferably 1. Materials sold under the Hostapur and Biosoft trademarks are examples of such anionic surfactants.

Still further examples of anionic surfactants include alkyl-diphenyl-ethersulphonates and alkyl-carboxylates. Other anionic surfactants can include salts (including, for example, sodium, potassium, ammonium, and substituted ammonium salts such as mono-, di-and triethanolamine salts) of soap, $C_6$-$C_{20}$ linear alkylbenzenesulfonates, $C_6$-$C_{22}$ primary or secondary alkanesulfonates, $C_6$-$C_{24}$ olefinsulfonates, sulfonated polycarboxylic acids prepared by sulfonation of the pyrolyzed product of alkaline earth metal citrates, $C_6$-$C_{24}$ alkylpolyglycolethersulfates, alkyl ester sulfates such

as $C_{14-16}$ methyl ester sulfates; acyl glycerol sulfonates, fatty oleyl glycerol sulfates, alkyl phenol ethylene oxide ether sulfates, paraffin sulfonates, alkyl phosphates, isethionates such as the acyl isethionates, N-acyl taurates, alkyl succinamates and sulfosuccinates, monoesters of sulfosuccinate (especially saturated and unsaturated $C_{12}$-$C_{18}$ monoesters) diesters of sulfosuccinate (especially saturated and unsaturated $C_6$-$C_{14}$ diesters), acyl sarcosinates, sulfates of alkylpolysaccharides such as the sulfates of alkylpolyglucoside, branched primary alkyl sulfates, alkyl polyethoxy carboxylates such as those of the formula $RO(CH_2CH_2O)_kCH_2COO^-M^+$ wherein R is a $C_8$-$C_{22}$ alkyl, k is an integer from 0 to 10, and M is a soluble salt-forming cation.

Desirably the inventive compositions necessarily comprise at least one anionic surfactant which necessarily which provides good foaming when used, and which is not undesirably irritating to the skin when topically applied. By way of non-limiting example suitable foaming anionic surfactants include, but are not limited to: alkyl sulfates, alkyl ether sulfates, alpha-olefin sulfonates, and mixtures thereof, particularly $C_{12}$-$C_{16}$ olefin sulfonates as well as salt forms thereof, e.g., sodium lauryl sulfate and ammonium lauryl sulfate. Such foaming anionic surfactants, particularly the preferred $C_{12}$-$C_{16}$ olefin sulfonates, exhibit high foaming and good lathering to the inventive compositions.

In certain particularly preferred embodiments, preferably at least one $C_{12}$-$C_{16}$ olefin sulfonate, which is preferably present as a salt such as an alkali or alkali earth metal salt such as sodium or potassium, or as an ammonium salt, and particularly preferably a lauryl sulfate or an ammonium lauryl sulfate, is present in conjunction with at least one $C_{12}$-$C_{16}$ ether sulfate, which is preferably present as a salt such as a sodium or ammonium salt, and particularly preferably a sodium lauryl ether sulfate, but more preferably both are necessarily present in the anionic surfactant constituent. In certain especially preferred embodiments the sole anionic surfactant constituent present is both at least one $C_{12}$-$C_{16}$ olefin sulfonate or salt thereof concurrently with at least one $C_{12}$-$C_{16}$ ether sulfate or salt thereof to the exclusion of other anionic surfactants.

In certain further particularly preferred embodiments, preferably at least one $C_{12}$-$C_{16}$ ether sulfate, which is preferably present as an alkali metal salt such as an alkali or alkaline earth metal salt such as sodium or potassium, or as an ammonium salt, and particularly preferably an ammonium salt of a lauryl ether sulfate is necessarily present as the anionic surfactant constituent. In certain especially preferred embodiments the sole anionic surfactant constituent present is one or more $C_{12}$-$C_{16}$ ether sulfates or salts thereof, especially ammonium salts, and particularly an ammonium salt of a lauryl ether sulfate to the exclusion of other anionic surfactants. An exemplary preferred $C_{12}$-$C_{16}$ ether sulfate which is commercially available is STANDAPOL EA-2, described by its supplier to be ammonium laureth-2 sulfate.

The one or more anionic surfactants are present in amounts of from 0.1 - 15 %wt., preferably in amounts from 0.5 - 15%wt., but are most desirably present in reduced weight percentages from about 1 - 12%wt. based on the total weight of the topical composition of which they form a part.

An optional but preferred additional constituent is a foam booster which improves the foaming characteristics of the anionic surfactant(s) present, but are not considered as anionic surfactants, per se. Preferred foam boosters are based on one or more alkanolamides which provide composition thickening, foam enhancement, and foam stability and in preferred embodiments of the invention are necessarily present. Exemplary alkanolamides which provide such a foam boosting function include but are not limited to: cocamide MEA, cocamide DEA, soyamide DEA, lauramide DEA, oleamide MIPA, stearamide MEA, myristamide MEA, lauramide MEA, capramide DEA, ricinoleamide DEA, myristamide DEA, stearamide DEA, oleylamide DEA, tallowamide DEA, lauramide MIPA, tallowamide MEA, isostearamide DEA, isostearamide MEA, and mixtures thereof. When present, the one ore more alkanolamides are present in amounts of up to about 10%wt., but are preferably included in amounts of from about 0.1- 8%wt. based on the total weight of the topical composition of which they form a part.

A particularly preferred surfactant constituent is a blend of materials commercially marketed as STEPANOL D-HS which is described by its supplier to be a blend of ammonium lauryl sulfate, sodium lauryl ether sulfate, and lauramide DEA which provides good foaming, and low irritancy to the skin.

In addition to the anionic surfactant constituent, the inventive compositions may include one or more further surfactants selected from nonionic and amphoteric (including betaine and zwitterionic) surfactants as well as mixtures thereof.

Exemplary useful nonionic surfactants are those which include a hydrophobicic base portion, such as a long chain alkyl group or an alkylated aryl group, and a hydrophilic chain portion comprising a sufficient number of ethoxy and/or propoxy moieties to render the nonionic surfactant at least partially soluble or dispersible in water. By way of non-limiting example, such nonionic surfactants include ethoxylated alkylphenols, ethoxylated and propoxylated fatty alcohols, polyethylene glycol ethers of methyl glucose, polyethylene glycol ethers of sorbitol, ethylene oxidepropylene oxide block copolymers, ethoxylated esters of fatty ($C_6$ -$C_{24}$) acids, condensation products of ethylene oxide with long chain amines or amides, and mixtures thereof. Further exemplary nonionic surfactants include, but are not limited to: methyl gluceth-10, PEG-20 methyl glucose distearate, PEG-20 methyl glucose sesquistearate, $C_{11}$-$C_{15}$ pareth-20, ceteth-8, ceteth-12, dodoxynol-12, laureth-15, PEG-20 castor oil, polysorbate 20, steareth-20, polyoxyethylene-10 cetyl ether, polyoxyethylene-10 stearyl ether, polyoxyethylene-20 cetyl ether, polyoxyethylene-10 oleyl ether, polyoxyethylene-20 oleyl ether, an ethoxylated nonylphenol, ethoxylated octylphenol, ethoxylated dodecylphenol, or ethoxylated fatty ($C_6$ -$C_{22}$) alcohol,

including 3 to 20 ethylene oxide moieties, polyoxyethyiene-20 isohexadecyl ether, polyoxyethylene-23 glycerol laurate, polyoxyethylene-20 glyceryl stearate, PPG-10 methyl glucose ether, PPG-20 methyl glucose ether, polyoxyethylene-20 sorbitan monoesters, polyoxyethylene 80 castor oil, polyoxyethylene-15 tridecyl ether, polyoxyethylene-6 tridecyl ether, laureth-2, laureth-3, laureth-4, PEG-3 castor oil, PEG 600 dioleate, PEG 400 dioleate, and mixtures thereof. Other nonionic surfactants, although not specifically disclosed herein but known to the art may also be used. The nonionic surfactants may be present as single compounds or as mixtures of two or more nonionic surfactant compounds.

Exemplary useful amphoteric surfactants include derivatives of secondary and tertiary amines having aliphatic radicals that are straight chain or branched, and wherein one of the aliphatic substituents contains from about 8 to 18 carbon atoms and at least one of the aliphatic substituents contains an anionic water-solubilizing group, e.g., a carboxy, sulfonate, or a sulfate group. Non-limiting examples of compounds falling within this description include: sodium 3-(dodecylamino)propionate, sodium 3-(dodecylamino)propane-1-sulfonate, sodium 2-(dodecylamino)ethyl sulfate, sodium 2-(dimethylamino)octadecanoate, disodium 3-(N carboxymethyldodecylamino)propane-1-sulfonate, disodium octadecyliminodiacetate, sodium 1-carboxymethyl-2-undecylimidazole, and sodium N,N-bis(2-hydroxyethyl)-2-sulfato-3-dodecoxypropylamine. Further exemplary useful amphoteric surfactants include sarcosinates and taurates, amide sulfosuccinates, and betaines including phosphobetaines. Further amphoteric surfactants, although not specifically elucidated herein but known to the art may also be used.

A particularly useful class of such further amphoteric surfactants are water-soluble betaine surfactants which may be represented by the general formula:

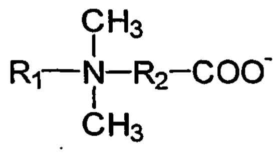

wherein $R_1$ is an alkyl group containing from 8 to 18 carbon atoms, or the amido radical which may be represented by the following general formula:

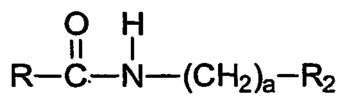

wherein R is an alkyl group having from 8 to 18 carbon atoms, a is an integer having a value of from 1 to 4 inclusive, and $R_2$ is a $C_1$-$C_4$ alkylene group. Examples of such water-soluble betaine surfactants which are advantageously used include dodecyl dimethyl betaine, cocoamidopropylbetaine and laurylamidopropyl betaine. An exemplary preferred water soluble betaine which is presently currently available is VELVETEX BK-35, described by its supplier to be primarily cocoamidopropyl betaine.

When included in the topical compositions of the invention either or both of the further optional nonionic or amphoteric surfactants may be present in amounts of from about from 0.1 - 15 %wt., preferably in amounts from 0.2 - 10%wt., but are most desirably present in reduced weight percentages from about 0.2 - 8%wt. based on the total weight of the topical composition of which they form a part.

In certain particularly preferred embodiments of the invention the surfactant constituent necessarily includes one or more water soluble betaine surfactants preferably in conjunction with one or more anionic surfactants, especially preferably one or more $C_{12}$-$C_{16}$ ether sulfate anionic surfactants or salts thereof as described previously.

In certain particularly preferred embodiments the surfactants present are one or more $C_{12}$-$C_{16}$ ether sulfate anionic surfactants or salts thereof concurrently with one or more water soluble betaine surfactants to the exclusion of at least further anionic and amphoteric surfactants. In certain especially preferred embodiments the sole surfactants present are one or more $C_{12}$-$C_{16}$ ether sulfate anionic surfactants or salts thereof concurrently with one or more water soluble betaine surfactants to the exclusion of

The topical compositions of the invention further necessarily comprise one or more emollients which provide softness to the compositions.

One class of useful emollients are cationic Polyquaternium-type polymers which are known to the art of topical compositions. Various grades of such cationic polymers may be used, inter alia: homopolymers of diallyldimethylammonium chloride commercially available as Polyquaternium 5; dimethyldiallyammonium chloride homopolymer commercially available as Polyquaternium 6; copolymers of diallyldimethylammonium chloride with acrylamide commercially available as Polyquaternium 7; polymeric quaternary ammonium salt derived from the reaction of hydroxyethyl cellulose

with a trimethylammonium substituted epoxide commerically available as polymeric quaternary ammonium salts derived from the reaction of hydroxyethyl cellulose with a trimethylammonium substituted epoxide commercially available as Polyquaternium 10; copolymers of 1-vinyl-2-pyrrolidine and dimethylaminoethyl methacrylate commercially available as Polyquaternium 11; Polyquaternium 15; copolymers of 1-vinyl-2-pyrrolidine and 1-vinyl-3-methylimidazolium salt commercially available as Polyquaternium 16; copolymers of acrylic acid and dimethyldiallylammonium chloride commercially available as Polyquaternium 22; polymeric quaternary ammonium salts of hydroxyethyl cellulose reacted with lauryl dimethyl ammonium-substituted epoxide commercially available as Polyquaternium 24; Polyquaternium 28; polymeric quaternary ammonium salts of the terpolymer of acrylic acid/diallyldimethylammonium chloride/acrylamide commercially available as Polyquaternium 39; Polyquaternium 44; Polyquaternium 46; and, terpolymers of acrylic acid with methacrylamidopropyl trimethylammonium chloride and methylacrylate commercially available as Polyquaternium 47. Other polyquaternium compounds although not specifically elucidated here may also be utilized in the present inventive compositions. When included in the inventive compositions, the one or more cationic polyquaternium-type polymers may be present in amounts of from about from 0.001 - 5 %wt., preferably in amounts from 0.01- 2.5%wt., but are most desirably present in reduced weight percentages from about 0.05 - 1%wt. based on the total weight of the topical composition of which they form a part.

Further emollients useful in the topical compositions include one or more of esters, fatty acids and alcohols, polyols and hydrocarbons which may impart a softening effect when topically applied. Exemplary esters include mono- and di-esters which may be, inter alia, dibutyl adipate, diethyl sebacate, diisopropyl dimerate, dioctyl succinate, Exemplarly branched chain fatty esters include 2-ethyl-hexyl myristate, isopropyl stearate and isostearyl palmitate. Exemplary tribasic acid esters include triisopropyl trilinoleate and trilauryl citrate. Exemplarly straight chain fatty esters include lauryl palmitate, myristyl lactate, oleyl eurcate and stearyl oleate. Further exemplary useful esters include coco-caprylate/caprate (a blend of coco-caprylate and coco-caprate), propylene glycol myristyl ether acetate, diisopropyl adipate and cetyl octanoate. Exemplary useful fatty alcohols and acids include, inter alia, those compounds having from 10 to 20 carbon atoms, preferentially cetyl, myristyl, palmitic and stearyl alcohols and acids. Exemplary useful polyols include, inter alia, linear and branched chain alkyl polyhydroxyl compounds such as, propylene glycol, sorbitol and glycerin. Further useful emollients include polymeric polyols, inter alia, polypropylene glycol and polyethylene glycol. Exemplary hydrocarbons which may serve as emollients are those having hydrocarbon chains anywhere from 12 to 30 carbon atoms, particularly, mineral oil, petroleum jelly, squalene and isoparaffins.

The emollient constituent may be a single compound or a mixture of two or more compounds which provide a beneficial emollient effect. The total amount of the emollient constituent(s) present are sufficient to provide an improved softening effect to the inventive compositions and are advantageously included in amounts of from 0.05 - 10%wt. based on the total weight of the emollient constituent(s) present in the composition. Desirably the emollient constituent(s) are included in the present inventive composition in amounts from 0.05 - 5%wt., but are most desirably present in reduced weight percentages from about 0.1 - 3%wt. based o n the total weight of the topical composition of which they form a part.

According to certain preferred embodiments the present compositions necessarily include at least one emollient selected from Polyquaternium-type polymers and alkyl polyhydroxyl compounds, e.g. glycerine. According to certain particularly preferred embodiments the emollient constituent comprises both a Polyquaternium-type polymers and alkyl polyhydroxyl compounds, especially glycerine which are provided in respective weight ratios of 1:5 to 1:20, preferably 1:8 - 1:15, and especially in respective weight ratios of 1:8 to 1:12.

In order to provide effective antimicrobial efficacy the present inventors have found that the most effective antimicrobial efficacy is provided by the inclusion of salicylic acid in an antimicrobially effective amount as the primary antimicrobial agent or constituent. The present inventors have surprisingly observed that in the preferred pH range of the topical compositions, salicylic acid exhibited surprisingly superior antimicrobial efficacy against both gram positive and gram negative microorganisms, and in particularly against both *S.aureus* and *E.coli* within specific pH ranges. While other organic acids, e.g., citric, tartaric, maleic, lactic, malic, benzoic, and the like provided some degree of antimicrobial efficacy such were either shown not to provide or are not expected to provide the same superior degree of antimicrobial effectiveness against both gram positive and gram negative microorganisms as provided by topical composition according to the invention wherein the primary antimicrobial agent is salicylic acid. It is also contemplated that in addition to or in place of the salicylic acid which is most preferred, salicylates (including octyl, amyl, phenyl, benzyl, menthyl, glyceryl, and dipropyleneglycol esters thereof) may also be included in the inventive compositions and are believed to be effective against both gram positive and gram negative microorganisms as well.

The inventors have also found that the superior antimicrobial efficacy is provided wherein, in addition to the use of salicylic acid as the primary antimicrobial agent, that the topical compositions are also maintained in a specific acidic pH range, preferably in the range of from about 2 - 6, yet more preferably from about 3.0 to about 5.0, and yet more preferably from about 3.7 to about 4.6, and most preferably from about 3.85 to about 4.6.

In view of these foregoing requirements the salicylic acid (and/or salicylates) is necessarily present in an amount effective to be antimicrobially effective against both gram positive and gram negative microorganisms and at the same time be included in sufficient amounts that in conjunction with any other acid constituent which may be present, that the

pH of the topical composition is in the specific pH range noted previously. Whereas the salicylic acid (and/or salicylates) is necessarily included as the primary antimicrobial agent, it is contemplated that one or more further acids may be present as co-acids in order to adjust the pH of the topical composition to a specific and advantageously to a preferred pH range. Such further co-acids may provide a secondary or ancillary antimicrobial benefit but are preferably primarily intended to be used in order to provide a pH adjustment. According to certain preferred embodiments the topical compositions of the invention include salicylic acid (and/or salicylates) in excess of any other organic acid or inorganic acid constituent which may be present, desirably if other organic acid or inorganic acid constituents are present, the salicylic acid (and/or salicylates) is/are present in at least three, preferably at least four times the amount of the total mass of other organic acid or inorganic acid constituents which may be present. According to certain particularly preferred embodiments the salicylic acid (and/or salicylates) are present in the absence of other organic acid or inorganic acid constituents.

While the salicylic acid (and/or salicylates) acid may be present in any effective amount in order to satisfy the foregoing requirements concerning antimicrobial efficacy and pH adjustment, advantageously the salicylic acid (and/or salicylates) is present in amount of from about 0.001 - 1%wt., preferably from about 0.001- 0.5%wt. and most preferably from about 0.01- 0.3%wt. The total amount of any other additional organic or inorganic acids which may be included to provide a pH adjustment effect and potentially a secondary antimicrobial effect desirably does not exceed one-half of the amount of the salicylic acid (and/or salicylates) as discussed previously, although in certain preferred embodiments such further additional organic or inorganic acids are absent or excluded from the topical compositions of the invention.

Surprisingly it was observed that the omission of salicylic acid, and the inclusion of certain other acids in a similar amount, specifically the substitution of the salicylic acid by a similar amount or like amount of lactic acid or citric acid did not provide the antimicrobial benefits provided by the presence of the salicylic acid in the topical compositions. Hence, the use of salicylic acid in the absence of additional organic or inorganic acids is both advantageous from both a cost and efficacy standpoint and is thus preferred.

The inventive compositions necessarily include one or more water-soluble inorganic or organic salt or mixtures of such salts. For purposes of the present invention, with reference to the organic s "water soluble" is to be understood as having a solubility in water of at least 10 grams per hundred grams of water at 20°C. Contemplated to be useful salts include various alkali metal and/or alkaline earth metal salts of sulfates, chlorides, borates, bromides, fluorides, phosphates, carbonates, bicarbonates, citrates, acetates, lactates, and the like. Specific examples of suitable salts include sodium sulfate, sodium chloride, potassium sulfate, sodium carbonate, lithium chloride, tripotassium phosphate, sodium borate, potassium bromide, potassium fluoride, sodium bicarbonate, calcium chloride, magnesium chloride, sodium citrate, sodium acetate, calcium lactate, magnesium sulfate and sodium fluoride. Such salts upon dissolution desirably interact with at least one but preferably with one or more of the surfactant constituents present in the topical composition in order to provide a thickening effect which would not be achieved absent the presence of the salt. It is contemplated that essentially any water soluble or water dispersible organic or inorganic salt or salts may be incorporated in the composition which is observed to provide such a thickening benefit while not undesirably detracting from the stability or antimicrobial efficacy of the topical compositions may be used. The preferred salts are the inorganic salts, especially the alkali metal sulfates and chlorides. Particularly preferred salts, because of its low cost and good efficacy is calcium chloride. The inorganic or organic salt or mixtures thereof are present in the topical compositions in any amount which is effective to increase the viscosity of the topical compositions. Advantageously the inorganic or organic salt or mixtures thereof are present in an amount of from 0.001- 5%wt., more preferably in amount of from 0.01 - 3.5%wt., and most preferably are present in an amount of from 0.05 - 1.2%wt. based on the total weight of the composition of which it forms a part. Alternately the inorganic or organic salt or mixtures thereof may be present in any amount which is effective to provide increased thickening of the topical compositions, desirably is present in an amount to assist in imparting a viscosity of at least about 500 cps, but preferably higher viscosities as described hereinafter. The inventors have also discovered that the use of sodium inorganic salts such as sodium chloride are to be avoided as it has been observed that the use of sodium salts may diminish the antimicrobial efficacy of the topical compositions against *E.coli* to an undesirable extent.

While the use of a further optional thickener constituent is contemplated, in certain particularly preferred embodiment such further optional thickeners such as those based on polymeric thickeners, clays, or celluloses are desirably absent from the topical compositions.

The compositions are at a neutral or acidic pH, but preferably is a pH in the range of from about 2 - 6, yet more preferably from about 3.0 to about 5.0, and yet more preferably from about 3.7 to about 4.6, and most preferably from about 3.85 to about 4.6. The pH of the topical compositions may be adjusted by utilization of an acid which may be any known art organic or inorganic acid which is useful in cosmetic or topical compositions to provide such a pH adjusting effect. Exemplary acids which may included in the topical compositions to provide a pH adjusting effect include mineral acids and polycarboxylic acids. Nonlimiting examples of useful mineral acids are hydrochloric acid, nitric acid, phosphoric acid, and sulfuric acid. Nonlimiting examples of polycarboxylic acids are citric acid, glycolic acid, and lactic acid. The acids may be provided to the other constituents in an anhydrous form, or may be provided as an aqueous solution or

dispersion or in an aqueous/organic solvent as a solution or dispersion to the other constituents used to produce the topical composition. It is contemplated that a single acid, or two or more acids may be used in combination to provide a pH adjusting effect. Generally only minor amounts of one or more acids are required to provide a pH adjusting effect in the topical compositions, and generally amounts required to adjust the pH do not exceed 3%wt., preferably do not exceed 2%wt. of the acid(s) based on the total weight of the topical composition. Citric acid, which is widely available at low cost but which is effective in adjusting the pH of the final compositions is preferred for use.

The inventive compositions include one or more chelating agents. Exemplary useful chelating agents include those known to the art, including by way of non-limiting example; aminopolycarboxylic acids and salts thereof wherein the amino nitrogen has attached thereto two or more substituent groups. Preferred chelating agents include acids and salts, especially the sodium and potassium salts of ethylenediaminetetraacetic acid, diethylenetriaminepentaacetic acid, N-hydroxyethylethylenediaminetriacetic acid, and of which the sodium salts of ethylenediaminetetraacetic acid may be particularly advantageously used. Such chelating agents may be omitted, or they may be included in generally minor amounts such as from 0 - 0.5 %wt. based on the weight of the chelating agents and/or salt forms thereof. Desirably, such chelating agents are included in the present inventive composition in amounts from 0 - 0.5%wt., but are most desirably present in reduced weight percentages from about 0 - 0.2%wt.

The topical compositions may include one or more further optional constituents which may be used to impart one or more desired esthetic or technical benefits to the topical compositions. In certain preferred embodiments of the invention, one or more of the following recited optional constituents may be considered as essential constituents according to a particular preferred embodiment. Such optional constituents include additives and adjuvants which are conventional in the cosmetic, pharmaceutical or dermatological field, such as hydrophilic or lipophilic gelling agents, hydrophilic or lipophilic active agents, opacifiers, preservatives, antioxidants, solvents especially including organic solvents as well as water or, fragrances, fillers, preservatives, dyestuffs or colorants, and light stabilizers including UV absorbers. The amounts of these various additives and adjuvants are those conventionally used in the field, and, for example, range from 0.01 % to 10% of the total weight of the composition.

One optional constituent which may be included in the inventive compositions, and in certain preferred embodiments is an essential constituent, is a latex. Such are used to provide opacification of the composition. Such are materials which are typically emulsions, dispersions or suspensions of a a water insoluble polymer or copolymer in an carrier. The carrier may be aqueous, an aqeueous/organic solvent mixture or organic solvent. The latex may be based on a homopolymer, or on copolymer. It is contemplated that the copolymer comprises two or more different monomers which are joined in either a block or random arrangement of the two or more different monomers.

Exemplary copolymers suitable for the latex emulsion include those formed from styrene, alpha-methylstyrene, divinylbenzene, acrylic acid, methacrylic acid, $C_1$ -$C_{20}$ esters of acrylic acid or methacrylic acid, acrylamide, methacrylamide, maleic acid, vinyl acetate, crotonic acid, vinyl neodecanoate and butenoic acid. Examples of carboxylate type copolymers are the styrene/alkyl acrylate and partially esterified polyacrylic and polymethacrylic salts and free acid forms. Among the foregoing materials are poly(butyl methacrylate), poly(methyl acrylate), poly(methyl methacrylate), poly(acrylic acid/$C_1$ - $C_{20}$ alkyl acrylate) andpoly(methacrylic acid/$C_1$ -$C_{20}$ alkyl methacrylate). These copolymers may be prepared by polymerization of the respective monomers by traditional oil-in-water or water-in-oil emulsion polymerization techniques. Alternatively, a pseudo latex may be prepared by esterification of preformed polymer with $C_1$ -$C_{20}$ alkanol. Average diameters of the dispersed polymer may range from about 0.001 micron to about 120 micron, preferably from about 0.01 micron to about 1 micron, optimally from about 0.1 micron to about 0.5 micron.

Number average molecular weight for polymers according to the present invention may range from about 1,000 to about 1,000,000, preferably from about 2,000 to about 500,000, optimally from about 5,000 to about 20,000.

A variety of techniques well-known in the art can be used to prepare latexes of water-insoluble polymer particles. These include, inter alia, batch, semi-continuous and seeded emulsion polymerization techniques.

Particularly preferred latexes useful in the present invention are latexes presently commercially available under the trademark ACUSOL (ex. Rohm & Haas Inc.). The latexes are characterized by pH of about 2 to about 3, having approximately 40% solids in water, with particle size of about 0.1 to about 0.5 micron. Specific ACUSOL. polymers include ACUSOL OP301 described as being a latex of a styrene/acrylate polymer, ACUSOL OP302 described as being a latex of a styrene/acrylate/divinylbenzene copolymer, ACUSOL OP303 described as being a latex of a styrene/acrylamide copolymer, ACUSOL OP305 described as being a latex of a styrene/PEG-10 maleate/nonoxynol-10 maleate/acrylate copolymer and a styrene/acrylate/PEG-10 dimaleate copolymer. Further preferred latexes useful in the present invention include those styrene/polyvinylpyrrolidone co-polymers and styrene/acrylic emulsions. Such include styrene/polyvinylpyrrolidone co-polymers which can be used include, for example, POLECTRON 430 (ex. ISP Technologies, Inc.), as well as sodium styrene/acrylate/divinyl-benzene co-polymer and ammonium nonoxynol-4 sulfate; sodium stytene/PEG-10 maleate/nonoxynol-10 maleate/acrylates co-polymer and ammonium nonoxynol-4 sulfate; styrene/acrylamide co-polymer and ammonium nonoxynol-4 sulfate; styrene/acrylates co-polymer and sodium lauryl sulfate and octoxynol-9; sodium styrene/acrylates co-polymer and sodium lauryl sulfate and tridecath-7; sodium methacrylate/styrene co-polymer and sodium lauryl sulfate and tridecath-7 and sodium lauryl diphenyloxide-disulfonate; and sodium

styrene/acrylates co-polymer (ex CSA, Inc., Greenville, S.C.).

When present in a composition, in accordance with certain of the preferred embodiments, the latex may be present in amounts of up to about 5%wt., preferably are present in amounts of from about 0.001%wt. to about 3%wt., preferably are present in amount from about 0.1%wt. to about 1.2%wt, and most preferably are present in amounts of from about 0.1%wt. to about 1%wt., based on the total weight of the topical composition of which it forms a part. Concurrently the amount of the of the water-insoluble polymer present in the latex may range from about 0.01 to about 90%, preferably from about 0.1 to about 60%, optimally from about 10 to about 50% by weight of the latex. The topical compositions may include one or more preservatives. Exemplary useful preservatives include compositions which comprise parabens, including methyl parabens and ethyl parabens, glutaraldehyde, formaldehyde, 2-bromo-2-nitropropoane-1,3-diol, 5-chloro-2-methyl-4-isothiazolin-3-one, 2-methyl-4-isothiazoline-3-one, and mixtures thereof. One exemplary composition is a combination 5-chloro-2-methyl-4-isothiazolin-3-one and 2-methyl-4-isothiazolin-3-one where the amount of either component may be present in the mixture anywhere from 0.001 to 99.99 weight percent, based on the total amount of the preservative. For reasons of availability, the most preferred preservative are those commercially available preservative comprising a mixture of 5-chloro-2-methyl-4-isothiazolin-3-one and 2-methyl-4-isothiazolin-3-one marketed under the trademark KATHON CG/ICP as a preservative composition (ex. Rohm and Haas Inc.). Further useful preservative compositions include KATHON CG/ICP II (ex. Rohm and Haas Inc.), PROXEL (ex. Zeneca), SUTTOCIDE A (ex. Sutton Laboratories) as well as TEXTAMER 38AD (ex. Calgon Corp.) When present the preservative is included in any amount found to be effective in retarding or inhibiting the grown of undesired microorganisms in the topical compositions, particularly during storage for several months at room temperature. When present in a composition, in accordance with certain of the preferred embodiments, the preservative composition is advantageously present in amounts of up to about 1.5%wt., preferably are present in amounts of from about 0.00001%wt. to about 0.5%wt., and most preferably is present in an amount of from about 0.0001%wt. to 0.1%wt. based on the total weight of the topical composition of which it forms a part.

The topical compositions may include one or more fillers in the form of powders. By way of non-limiting examples these powders include chalk, talc, kaolin, starch, smectite clays, chemically modified magnesium aluminum silicate, organically modified montmorillonite clay, hydrated aluminum silicate, fumed silica, aluminum starch octenyl succinate and mixtures thereof. When present in a composition, in accordance with certain of the preferred embodiments, the latex may be present in amounts of up to about 5%wt., preferably are present in amounts of from about 0.001%wt. to about 5%wt. based on the total weight of the topical composition of which it forms a part.

The topical compositions may include a fragrance constituent, which may be based on natural and synthetic fragrances and most commonly are mixtures or blends of a plurality of such fragrances, optionally in conjunction with a carrier such as an organic solvent or a mixture of organic solvents in which the fragrances are dissolved, suspended or dispersed. By way of non-limiting example, natural fragrances include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamon, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, alpha-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams.

However, it is preferred to use mixtures of different perfume compounds which, together, produce an agreeable fragrance. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, labolanum oil and lavendin oil. When present in a composition, in accordance with certain of the preferred embodiments, the fragrance constituent may be present in any effective amount such that it can be discerned by a consumer of the topical composition, however is advantageously present in amounts of up to about 0.5%wt., preferably are present in amounts of from about 0.00001%wt. to about 0.2%wt., and most preferably is present in an amount of from about 0.0001%wt. to 0.1%wt. based on the total weight of the composition of which it forms a part.

The inventive compositions may include one or more colorants, e.g, dyes or pigments which are known to the art be useful in cosmetic or topical compositions which may be used to impart a desired color or tint to the inventive compositions. Any colorant which is compatible with the other constituents forming the topical compositions may be

used and such may be present in any amount effective to achieved the desired visual effect. Exemplary colorants include pigments, inter alia, inorganic red pigments, such as iron oxide, iron hydroxide and iron titanate; inorganic brown pigments, such as .gamma.-iron oxide; inorganic yellow pigments, such as iron oxide yellow and loess; inorganic black pigments, such as iron oxide black and carbon black; inorganic violet pigments, such as manganese violet and cobalt violet; inorganic green pigments, such as chromium hydroxide, chromium oxide, cobalt oxide and cobalt titanate; inorganic blue pigments, such as Prussian blue and ultramarine blue; lakes of tar pigments; lakes of natural dyes; and synthetic resin powder complexes of the inorganic pigments as recited above. Advantageously one or more colorants may be added in amounts of about 0.001%wt. to about 0.1% by weight, based on the total weight of the composition of which the colorant(s) forms a part.

The topical compositions may comprise one or more humectants, including polyhydric alcohols including polyalkylene glycols as well as alkylene polyols and their derivatives, inter alia, including propylene glycol, dipropylene glycol, polypropylene glycol, polyethylene glycol and derivatives thereof, sorbitol, hydroxypropyl sorbitol, erythritol, threitol, pentaerythritol, xylitol, glucitol, mannitol, hexylene glycol, butylene glycol (e.g., 1,3-butylene glycol), hexane triol (e.g., 1,2,6-hexanetriol), glycerine, ethoxylated glycerine and propoxylated glycerine. Further useful humectants include sodium 2-pyrrolidone-5-carboxylate, guanidine; glycolic acid and glycolate salts (e.g. ammonium and quaternary alkyl ammonium); lactic acid and lactate salts (e.g. ammonium and quaternary alkyl ammonium); aloe vera in any of its variety of forms (e.g., aloe vera gel); hyaluronic acid and derivatives thereof (e.g., salt derivatives such as sodium hyaluronate); lactamide monoethanolamine; acetamide monoethanolamine; urea; and, panthenol. The humectants may be used singly or two or more humectants may be included in topical compositions of the invention. Of the humectants, aloe vera in one or more of its forms is preferred as being a naturally derived product. When present, in accordance with certain of the preferred embodiments, one or more humectants may be included in effective amounts, advantageously from 0.01 - 2.5%wt., preferably from 0.01 - 0.8%wt. based on the total weight of the composition of which it forms a part.

The topical compositions may optionally comprise one or more further cosmetic constituents which may provide a cosmetic or dermatological treatment benefit to the user of the topical composition. It is expected that any of a wide variety of additives including one or more of those known to the art to provide a cosmetic or dermatological treatment benefit may be used. The topical compositions may include one or more constituents, particularly essential oils which are selected to provide a so-called "aromatherapy benefit" to the user. Essential oils are complex mixtures of different organic molecules, such as terpenes, alcohols, esters, aldehydes, ketones and phenols. Such essential oils are frequently extracted from naturally occurring botanical sources such as flowers, stems, leaves, roots and barks of aromatic plants. While essential oils may be used singly, it is also common to utilize blends of essential oils in order to provide a conjunctive aroma benefit, and possibly a therapeutic benefit as well.

A variety of essential oils providing an aromatherapy benefit may be incorporated into the topical compositions of the invention either as a single essential oil or as a mixture of two or more essential oils. It is also to be recognized when used, an essential oil providing an aromatherapy benefit may replace all or part of any further fragrance constituent including the fragrance constituents discussed above as many of the essential oils providing an aromatherapy benefit are pungent and odiferous. Such essential oils providing an aromatherapy benefit may be used singly, as blends or mixtures of essential oils, or in combination with other fragrancing constituents which may be synthetically produced or naturally derived, but need not be derived from or contain essential oils per se. Frequently, due to their potency, essential oils are often supplied dispersed in a liquid carrier such as in one or more organic solvents in which the essential oils are dissolved or dispersed.

By way of non-limiting example, exemplary useful essential oils providing an aromatherapy benefit which may find use in the topical compositions of the invention include: Abies Sibirica oil, Amyris Balsamifera oil, Anise oil, Balm Mint oil, Basil oil, Bay oil, Bee Balm oil, Bergamot oil, Birch oil, Bitter Orange oil, Cabbage Rose oil, Calendula Officinalis oil, California Nutmeg oil, Camellia Sinensis oil, Capsicum Frutescers oleoresin, Caraway Oil, Cardamon Oil, Cedarwood Oil, Chamaecyparis Obtusa Oil, Chamomile Oil, Cinnamon Oil, Citronella Oil, Clary Oil, Clove Oil, Cloveleaf Oil, Coriander Oil, Coriander Seed Oil, Cyperus Esculentus Oil, Cypress Oil, Eucalyptus Citriodora Oil, Eucalyptus Globulus Oil, Fennel Oil, Gardenia Florida Oil, Geranium Maculatum Oil, Ginger Oil, Grapefruit Oil, Hops Oil, Hypericum Perforatum Oil, Hyptis Suaveolens Oil, Indigo Bush Oil, Jasmine Oil, Juniperus Communis Oil, Juniperus Virginiana Oil, Labdanum Oil, Laurel Oil, Lavandin Oil, Lavender Oil, Lemon Oil, Lemongrass Oil, Leptospermum Scoparium Oil, Lime Oil, Linden Oil, Litsea Cubeba Oil, Lovage Oil, Mandarin Orange Oil, Massoy Bark Oil, Matricaria Oil, Moroccan Chamomile Oil, Musk Rose Oil, Myrrh Oil, Myrtle Oil, Norway Spruce Oil, Nutmeg Oil, Olax DissitifloraOil, Olibanum, Opoponax Oil, Orange Flower Oil, Orange Oil, Palmarosa Oil, Parsley Seed Oil, Passionflower Oil, Patchouli Oil, Pelargonium Graveolens Oil, Peppermint Oil, Pine Oil, Pine Tar Oil, Pine Kernel Oil, Pine Oil, Pine Cone Oil, Rosemary Oil, Rose Oil, Rosewood Oil, Rue Oil, Sage Oil, Sambucus Nigra Oil, Sandalwood Oil, Sandarac Gum, Sassafras Officinale Oil, Sisymbrium Ino Oil, Spearmint Oil, Sweet Marjoram Oil, Sweet Violet Oil, Tar Oil, Thyme Oil, Vetiveria Zizanoides Oil, Wild Mint Oil, Ximenia Americana Oil, Yarrow Oil, Ylang Yang Oil, or any combinations thereof.

Preferred essential oils providing an aromatherapy benefit for use in the topical compositions of the present invention include one or more selected from chamomile oil, lavendin oil, lavender oil, grapefruit oil, lemon oil, line oil,

mandarin orange oil, orange flower oil and orange oil. Chamomile oil may be used to promote both a fresh, clean and attractive scent and possibly provide a stress-relaxing benefit to the user of the topical composition. Lavender oil, and lavendin, may be used to promote both a fresh and attractive scent and possibly also provide a stress-relaxing benefit to the user of the topical composition. One or more of grapefruit oil, lemon oil, line oil, mandarin orange oil, orange flower oil and orange oil provide a clean citrus scent and may possibly impart a perceived therapeutic benefit as well when used.

As used in the present invention, these one or more essential oils providing an aromatherapy benefit are present in an amount about 0.00001 wt. % to about 1 wt. %, based on the total weight of the composition. Preferably, the one or more essential oils providing an aromatherapy benefit are present in an amount about 0.00005 wt. % to about 0.75 wt. %, and more preferably about 0.0001 wt. % to about 0.5 wt. % of the total weight of the composition. It is to be understood that these one or more essential oils providing an aromatherapy benefit may be used with our without the optional fragrancing constituent recited previously and may be used wholly or partially in place of said fragrancing constituent.

The topical compositions may include one or more antioxidant constituents. Examples of antioxidants include but are not limited to, water-soluble antioxidants such as sulfhydryl compounds and their derivatives (e.g., sodium metabisulfite and N-acetylcysteine), lipoic acid and dihydrolipoic acid, resveratrol, lactoferrin, glutathione, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids, tocopherols e.g., tocopherol acetate, tocotrienols, and ubiquinone. Natural extracts containing antioxidants suitable for use in the topical compositions of this invention, include but not limited to, extracts containing flavonoids and isoflavonoids and their derivatives, extracts containing resveratrol and the like. Examples of such natural extracts include grape seed, green tea, pine bark, propolis, and the like. When present the total amount of such antioxidants are usually not in excess of 5%wt, preferably are present in amounts of from 0.0001 - 4%wt. based on the total weight of the topical composition of which it forms a part. In certain preferred embodiments an one or more antioxidants constituents are necessarily present.

Optionally the topical compositions may include one or more vitamins. Examples of vitamins which can be added include vitamin A, such as vitamin A oil, retinol, retinyl acetate and retinyl palmitate; vitamin B, including vitamin $B_2$ such as riboflavin, riboflavin butyrate and flavin adenine nucleotide, vitamin $B_6$ such as pyridoxine hydrochloride, pyridoxine dioctanoate and pyridoxine tripalmitate, vitamin $B_{12}$ and its derivatives, and vitamin $B_{15}$ and its derivatives; vitamin C, such as L-ascorbic acid, L-ascorbic acid dipalmitic ester, sodium (L-ascorbic acid)-2-sulfate and dipotassium L-ascorbic acid diphosphate; vitamin D, such as ergocalciferol and cholecarciferol; vitamin E, such as alpha-tocopherol, beta-tocopherol, gamma-tocopherol, dl-alpha-tocopheryl acetate, dl-alpha-tocopheryl nicotinate and dl-alpha-tocopheryl succinate. When present, in accordance with certain of the preferred embodiments, one or more vitamins may be included in effective amounts, advantageously from 0.0001- 1%wt., preferably from 0.001- 0.5%wt. based on the total weight of the composition of which it forms a part.

The topical compositions may include a milk protein compound and/or a milk protein hydrolysate which may be used as a skin moisturizer. Exemplary useful milk protein compounds include hydrolyzed milk protein, hydrolyzed milk protein derivative, or any combinations thereof. Suitable hydrolyzed milk protein derivatives include, for example, palmitoyl derivative and quaternary ammonium salt derivative. Casein compounds and extracts, as well as phosphoproteins obtained from dairy milk may be used as milk protein compounds as well. The milk protein compound and/or a milk protein hydrolysate may be presently singly or as a mixture of such compounds. When present, in accordance with certain of the preferred embodiments, the one or more milk protein compound and/or a milk protein hydrolysates may be included in effective amounts, advantageously from 0.0001 -1.5%wt., preferably from 0.001- 0.5%wt., and most preferably from 0.001- 0.25%wt. based on the total weight of the topical composition of which it forms a part.

Further extracts from plants may be used which however are not intended to provide a primary aromatherapy benefit or fragrancing benefit. One such extract is cotton milk which is derived from the cotton plant and is believed to provide beneficial benefits including moisturising, softening and anti-inflammatory therapeutic properties due to its high concentration of flavonoids and softening essential oil. When present, in accordance with certain of the preferred embodiments, such further plant extracts especially cotton milk may be included in effective amounts, advantageously from 0.0001- 5%wt., preferably from 0.001- 3%wt. based on the total weight of the topical composition of which it forms a part.

The topical compositions may include one or more light stabilizers as well as UV absorbers. Such materials are known to be useful in cosmetic or topical compositions and impart a degree of stability to the compositions which may comprise one or more components which may be deleteriously affected when exposed to certain sources of light, e.g., sunlight, fluorescent light sources. Other such materials are known to stabilize or improve the effect of colorants which may be present in the compositions. Any cosmetically acceptable material or compound which provides protection for one or more of the constituents in the inventive compositions from photolytic degradation or photooxidative degradation may be used.

Exemplary light stabilizers as well as UV absorbers include:

triazines including s-triazine, triazine derivatives e.g. 2,4,6-trianilino-(p-carbo-2'-ethyl-1'-hexyloxy)-1,3,5-triazine, anisotriazine, ethylhexyltriazone, diethylhexylbutamidotriazone, 2,4,6-tris(diisobutyl 4'-aminobenzalmalonate)-s-triazine and octyltriazone;
benzotriazoles and derivatives, e.g. drometrizole trisiloxane, methylenebis(benzotriazolyl)tetramethylbutylphenol;
benzophenone compounds and derivatives e.g., 2-hydroxy-4-methoxy benzophenone, 2-hydroxy-4-methoxy-4'-methyl benzophenone, n-hexyl 2-(4-diethylamino-2-hydroxybenzoyl)benzoate and 2,2'-dihydroxy-4-methoxy benzophenone as well as those materials currently marketed under the UVINUL tradename by BASF;
sulphonic acid derivatives of benzophenones, e.g., 2-hydroxy-4-methoxybenzophenon-5-sulphonic acid;
esters of benzalmalonic acid, e.g., 4-methoxy benzmalonic acid 2-ethylhexyl esters of benzalmalonic acid;
benzoxazole derivatives, e.g., 2,4-*bis*[5-1(dimethylpropyl)benzoxazol-2-yl(4-phenyl)imino]-6-(2-ethylhexyl)imino-1,3,5-triazine;
sulphonic acid derivatives of 3-benzylidencamphen, e.g. 4-(2-oxo-3-bomylidenmethyl)-benzene sulphonic acid and 2-methyl-5-(2-oxo-bornyliden) sulphonic acid;
cinnamic acid and cinnamic acid amides, esters of cinnamonic acid, e.g., ethylhexyl methoxycinnamate , isopropyl methoxycinnamate, isoamyl methoxycinnamate, DEA methoxycinnamate, diisopropyl methylcinnamate, glyceryl ethylhexanoate dimethoxycinnamate, 4-methoxy cinnamonic acid 2-ethylhexylester, 4-methoxy cinnamonic acid propylester, 4-methoxy cinnamonic acid isoamylester, 2-cyano-3,3-phenyl cinnamonic acid 2-ethythexylester (octocrylene);
propane-1,3-diones, e.g. 1-(4-tert.-butylphenyl)-3-(4'-methoxy-phenyl)-propane-1,3-dion;
phenylbenzimidazoles and sulfonated benzimidazoles, e.g., 2-phenylbenzimidazol-5-sulphonic acid, disodium phenyl dibenzimidazole tetrasulfonate;
salicylic acid derivatives including esters of salicylic acid, e.g., ethylhexyl salicylate, dipropylene glycol salicylate, TEA salicylate, salicylic acid 2-ethylhexylester, salicylic acid 4-isopropyl benzylester, salicylic acid homomenthylester;
compounds or derivatives of compounds based on benzylidenecamphor, e.g., 3-benzyliden camphor, 3-benzylidene norcamphor, 4-methylbenzylidenecamphor, benzylidenecamphorsulfonic acid, camphor benzalkonium methosulfate, terephthalylidenedicamphorsulfonic acid, polyacrylamidomethylbenzylidenecamphor and derivatives thereof;
4-aminobenzoic acid and derivatives e.g., 4-(dimethylamino) benzoic acid 2-ethylhexylester, 4-(dimethylamino) benzoic acid 2-octylester and 4-(dimethylamino) benzoic acid amylester:, Any of the foregoing materials provided as acids may used in free acid form or as a salt thereof, e.g., an alkali, alkaline earth, ammonium, alkylammonium, alkanolammonium salt form thereof.

Exemplary and preferred such materials which are presently commercially available include one or more of: CIBAFAST H liquid, described to be sodium benzotriazolyl butylphenol sulfonate with Buteth-3 and tributyl citrate; TINOGARD HS described to be sodium benzotriazolyl butylphenol sulfonates; TINOGARD AS described to be bumetrizole; TINOGARD TL described to be benzotriazolyl dodecyl p-cresol; and TINOGARD Q described to be tris(tetramethylhydroxypiperidinol) citrate. All of these foregoing materials are presently commercially available from Ciba Specialty Chemicals (Muttenz, CH.).
When present, the one or more light stabilizers as well as UV absorbers may be included in any effective amount.
Further optional constituents, although not particularly elucidated herein may also be included in effective amounts as may be deemed appropriate or necessary.
As is noted above, the compositions according to the invention are aqueous in nature. Water is added to order to provide to 100% by weight of the compositions of the invention. The water may be tap water, but is preferably distilled and is most preferably deionized water or "soft" water. If the water is tap water, it is preferably substantially free of any undesirable impurities such as organics or inorganics, especially minerals salts which are present in hard water which may thus undesirably interfere with the operation of the constituents present in the topical compositions according to the present invention. Water forms a major proportion of the inventive compositions and is necessarily present in amounts of at least 50%wt., preferably in amounts of at least 60%wt., and more preferably are present in amounts of at least 70%wt. based on the total weight of the topical composition of which it forms a part.
The compositions of the invention are viscous and exhibit a viscosity of at least 500 cps at room temperature as measured using a Brookfield viscometer, Type 3 spindle. Preferably the compositions exhibit viscosities in the range of at least about 500 cps as measured under these conditions. Yet more preferably the topical compositions of the invention exhibit a viscosity in the range of about 1000 to about 10,000 cps, yet more preferably from about 1500 to 7000 cps, and especially preferably from about 1500 - 4500 cps.
While the topical compositions disclosed herein find a primary use in application to the skin to provide a cleaning and an antimicrobial benefit and is contemplated as being provided in a dispenser for use in such a treatment, it is to be understood that this is not to be understood as a limiting definition and that other forms and other uses of the present inventive composition, such as face lotion, milky lotion, cream, face cleansing cream, massage materials, liquid toilet soap, as well as in hair care products such as shampoo, rinse or other hair or scalp treatment are expressly contemplated

as being within the scope of the present invention. It is to be further expressly understood that topical application of the topical composition disclosed herein may be applied to the skin on any part of the body, including the skin on the face, neck, chest, back, arms, axilla, hands, legs, and scalp.

The topical composition of the invention can be formulated as a lotion, a cream or a gel. The composition can be packaged in a suitable container to suit its viscosity and intended use by the consumer. For example, a lotion or cream can be packaged in a bottle, or a propellant-driven aerosol device or a container fitted with a pump suitable for finger operation. When the composition is a cream, it can simply be stored in a non-deformable bottle or squeeze container, such as a tube or a lidded jar. The invention accordingly also provides a closed container containing a cosmetically acceptable composition as herein defined.

The following examples below illustrate exemplary formulations as well as preferred embodiments of the invention. It is to be understood that these examples are provided by way of illustration only and that further useful formulations falling within the scope of the present invention and the claims may be readily produced by one skilled in the art without deviating from the scope and spirit of the invention.

## Examples

A number of topical compositions produced according to the invention described below were produced in according to the following general protocol:

Into a suitably sized vessel open to the atmosphere, a measured amount of room temperature water was provided after which the constituents were added in the following sequence: surfactants, salicylic acid, glycerine, Polyquaternium, followed by the remaining constituents which were added in any order, except for the inorganic salt (calcium chloride) which was added last to the composition as its addition promoted thickening of the compositions to a viscosity of from about 1800 - 5000 cps measured on a Brookfield viscometer, No. 3 spindle at room temperature. Mixing, which generally lasted from 5 minutes to 60 minutes was maintained until the particular formulation appeared to be homogeneous. The exemplary compositions were viscous but pourable, and retained well mixed characteristics (i.e., stable mixtures) upon standing. While the above sequence of addition of the individual constituents were followed it is to be noted that the constituents might be added in any other order, although it is preferred that the inorganic salt constituent be the last constituent to be added as its interaction with the surfactant constituent initiates thickening of the topical compositions. Subsequently when and if required a minor amount of an aqueous solution of citric acid was added to the compositions in order to adjust their pH to a specific or target value, usually in the range of from 4 and 5.5. In each of the compositions was added deionized water in "quantum sufficient" (q.s.) in order to provide 100 parts by weight of the specific composition

| **Example Formulation 1** | | |
|---|---|---|
| | **% W/W** | **%W/W** |
| **Constituent** | **"as supplied"** | **active weight basis** |
| deionized water | q.s. | q.s. |
| STEPANOL D-HS | 24.0 | 8.88 |
| salicylic acid | 0.10 | 0.1 |
| glycerin (USP grade) | 2.00 | 2.0 |
| Polyquaternium 7 | 0.20 | 0.2 |
| tetrasodium EDTA | 0.10 | 0.1 |
| KATHON CG | 0.02 | 0.00033 |
| ACUSOL OP301 | 0.20 | 0.076 |
| calcium chloride | 0.50 | 0.40 |
| perfume (proprietary composition) | 0.22 | 0.22 |
| aloe vera* | 0.10 | 0.1* |
| milk protein* | 0.10 | 0.1* |

(continued)

| Example Formulation 1 | | |
|---|---|---|
| | % W/W | %W/W |
| Constituent | "as supplied" | active weight basis |
| **Total** | **100.00** | **100.00** |
| * = indicates a preparation "as supplied" from the respective supplier, actual active weight of identified constituent not specified by supplier | | |

| Example Formulation 1A | | |
|---|---|---|
| | %W/W | %W/W |
| Constituent | "as supplied" | active weight basis |
| deionized water | q.s. | q.s. |
| STEPANOL D-HS | 24.0 | 8.88 |
| salicylic acid | 0.10 | 0.1 |
| glycerin (USP grade) | 2.00 | 2.0 |
| Polyquaternium 7 | 0.20 | 0.2 |
| tetrasodium EDTA | 0.10 | 0.1 |
| KATHON CG | 0.02 | 0.00033 |
| ACUSOL OP305 | 0.25 | 0.095 |
| calcium chloride | 0.50 | 0.40 |
| perfume (proprietary composition) | 0.22 | 0.22 |
| aloe vera* | 0.10 | 0.1* |
| milk protein* | 0.10 | 0.1* |
| **Total** | **100.00** | **100.00** |
| * = indicates a preparation "as supplied" from the respective supplier, actual active weight of identified constituent not specified by supplier | | |

| Example Formulation 1B | | |
|---|---|---|
| | %W/W | %W/W |
| Constituent | "as supplied" | active weight basis |
| deionized water | q.s. | q.s. |
| STANDAPOL EA2 | 27.0 | 6.75 |
| VELVETEX BK-35 | 7.5 | 2.25 |
| salicylic acid | 0.10 | 0.1 |
| glycerin (USP grade) | 2.00 | 2.0 |
| Polyquaternium 7 | 0.20 | 0.2 |
| tetrasodium EDTA | 0.10 | 0.1 |
| KATHON CG | 0.02 | 0.00033 |
| ACUSOL OP305 | 0.25 | 0.095 |

(continued)

| Example Formulation 1B | | |
|---|---|---|
| | %W/W | %W/W |
| Constituent | "as supplied" | active weight basis |
| calcium chloride | 0.30 | 0.25 |
| perfume (proprietary composition) | 0.22 | 0.22 |
| aloe vera* | 0.10 | 0.1* |
| milk protein* | 0.10 | 0.1* |
| **Total** | **100.00** | **100.00** |
| pH = 3.71 | | |
| Viscosity = 3413 cPs | | |
| * = indicates a preparation "as supplied" from the respective supplier, actual active weight of identified constituent not specified by supplier | | |

The foregoing compositions may have included an additional minor amount of citric acid in order to adjust the pH of each composition to a pH in the range of 3.7 to 4.0. Each of the foregoing compositions exhibited a viscosity of between 1500 - 4500 cPs, measured using a Brookfield viscometer, LV spindle #3, at 12 rpm at room temperature (approx. 20-22°C). The foregoing compositions provide antimicrobial efficacy when topically applied, while the inclusion of both aloe vera and milk protein provides a skin moisturizing benefit.

| Example Formulation 2 | | |
|---|---|---|
| | %W/W | %W/W |
| Constituent | "as supplied" | active weight basis |
| deionized water | q.s. | q.s. |
| STEPANOL D-HS | 24.00 | 8.88 |
| salicylic acid | 0.10 | 0.1 |
| glycerin (USP grade) | 2.00 | 2.0 |
| Polyquaternium 7 | 0.20 | 0.2 |
| tetrasodium EDTA | 0.10 | 0.1 |
| KATHON CG | 0.02 | 0.00033 |
| ACUSOL OP301 | 0.20 | 0.076 |
| calcium chloride | 0.50 | 0.40 |
| perfume (proprietary composition) | 0.20 | 0.20 |
| cotton milk* | 0.10 | 0.1* |
| chamomile extracts* | 0.10 | 0.1* |
| colorant 1 (proprietary composition) | 0.00010 | 0.0001 |
| colorant 2 (proprietary composition) | 0.00030 | 0.0003 |
| **Total** | **100.00** | **100.00** |
| * = indicates a preparation "as supplied" from the respective supplier, actual active weight of identified constituent not specified by supplier | | |

| Example Formulation 2A | | |
|---|---|---|
| | %W/W | %W/W |
| Constituent | "as supplied" | active weight basis |
| deionised water | q.s. | q.s. |
| STEPANOL D-HS | 24.00 | 8.88 |
| salicylic acid | 0.10 | 0.1 |
| glycerin (USP grade) | 2.00 | 2.0 |
| Polyquaternium 7 | 0.20 | 0.2 |
| tetrasodium EDTA | 0.10 | 0.1 |
| KATHON CG | 0.02 | 0.00033 |
| ACUSOL OP301 | 0.20 | 0.076 |
| calcium chloride | 0.50 | 0.40 |
| perfume (proprietary composition) | 0.20 | 0.20 |
| cotton milk* | 0.10 | 0.1* |
| chamomile extract* | 0.10 | 0.1* |
| CIBAFAST H Liquid | 0.10 | 0.03 |
| colorant 1 (proprietary composition) | 0.00010 | 0.0001 |
| colorant 2 (proprietary composition) | 0.00030 | 0.0003 |
| **Total** | **100.00** | **100.00** |
| * = indicates a preparation "as supplied" from the respective supplier, actual active weight of identified constituent not specified by supplier | | |

| Example Formulation 2B | | |
|---|---|---|
| | %W/W | %W/W |
| Constituent | "as supplied" | active weight basis |
| deionised water | q.s. | q.s. |
| STANDAPOL EA2 | 27.00 | 6.75 |
| VELVETEX BK-35 | 7.5 | 2.25 |
| salicylic acid | 0.10 | 0.1 |
| glycerin (USP grade) | 2.00 | 2.0 |
| Polyquaternium 7 | 0.20 | 0.2 |
| tetrasodium EDTA | 0.10 | 0.1 |
| KATHON CG | 0.02 | 0.00033 |
| ACUSOL OP301 | 0.20 | 0.076 |
| calcium chloride | 0.30 | 0.25 |
| perfume (proprietary composition) | 0.20 | 0.20 |
| cotton milk* | 0.10 | 0.1* |
| chamomile extract* | 0.10 | 0.1* |
| CIBAFAST H Liquid | 0.10 | 0.03 |

(continued)

| Example Formulation 2B | | |
|---|---|---|
| | %W/W | %W/W |
| Constituent | "as supplied" | active weight basis |
| colorant 1 (proprietary composition) | 0.00010 | 0.0001 |
| colorant 2 (proprietary composition) | 0.00030 | 0.0003 |
| **Total** | **100.00** | **100.00** |
| pH = 3.71 | | |
| Viscosity = 2773 cPs | | |
| * = indicates a preparation "as supplied" from the respective supplier, actual active weight of identified constituent not specified by supplier | | |

The foregoing compositions may have included an additional minor amount of citric acid in order to adjust the pH of each composition to a pH in the range of 3.7 to 4.0. Each of the foregoing compositions exhibited a viscosity of between 1500 - 4500 cPs, measured using a Brookfield viscometer, LV spindle #3, at 12 rpm at room temperature (approx. 20-22°C). The foregoing compositions provide antimicrobial efficacy when topically applied while the inclusion of both chamomille and milk protein provides a skin moisturizing effect and a calming, aromatherapy benefit.

| Example Formulation 3 | | |
|---|---|---|
| | %W/W | %W/W |
| Constituent | "as supplied" | active weight basis |
| deionized water | q.s. | q.s. |
| STEPANOL D-HS | 24.00 | 8.88 |
| salicylic acid | 0.10 | 0.1 |
| glycerin (USP grade) | 2.00 | 2.0 |
| Polyquaternium 7 | 0.20 | 0.2 |
| tetrasodium EDTA | 0.10 | 0.1 |
| KATHON CG | 0.02 | 0.00033 |
| calcium chloride | 0.50 | 0.40 |
| perfume (proprietary composition) | 0.20 | 0.20 |
| grapefruit extracts* | 0.10 | 0.10* |
| Vitamin E acetate* | 0.20 | 0.20* |
| colorant 1 (proprietary composition) | 0.00035 | 0.00035 |
| colorant 2 (proprietary composition) | 0.00015 | 0.00015 |
| **Total** | **100.00** | **100.00** |
| * = indicates a preparation "as supplied" from the respective supplier, actual active weight of identified constituent not specified by supplier | | |

| Example Formulation 3A | | |
|---|---|---|
| | % W/W | %W/W |
| Constituent | "as supplied" | active weight basis |
| deionized water | q.s. | q.s. |

(continued)

| Example Formulation 3A | | |
|---|---|---|
| | % W/W | %W/W |
| **Constituent** | **"as supplied"** | **active weight basis** |
| STEPANOL D-HS | 24.00 | 8.88 |
| salicylic acid | 0.10 | 0.1 |
| glycerin (USP grade) | 2.00 | 2.0 |
| Polyquaternium 7 | 0.20 | 0.2 |
| tetrasodium EDTA | 0.10 | 0.1 |
| KATHON CG | 0.02 | 0.00033 |
| calcium chloride | 0.75 | 0.60 |
| perfume (proprietary composition) | 0.20 | 0.20 |
| grapefruit extracts* | 0.10 | 0.10* |
| Vitamin E acetate* | 0.20 | 0.20* |
| colorant 1 (proprietary composition) | 0.00006 | 0.00006 |
| colorant 2 (proprietary composition) | 0.0004 | 0.0004 |
| CIBAFAST H Liquid | 0.30 | 0.09 |
| TINOGARD Q | 0.30 | 0.03 |
| **Total** | **100.00** | **100.00** |
| * = indicates a preparation "as supplied" from the respective supplier, actual active weight of identified constituent not specified by supplier | | |

| Example Formulation 3B | | |
|---|---|---|
| | %W/W | %W/W |
| **Constituent** | **"as supplied"** | **active weight basis** |
| deionized water | q.s. | q.s. |
| STANDAPOL EA2 | 27.00 | 6.75 |
| VELVETEX BK-35 | 7.5 | 2.25 |
| salicylic acid | 0.10 | 0.1 |
| glycerin (USP grade) | 2.00 | 2.0 |
| Polyquaternium 7 | 0.20 | 0.2 |
| tetrasodium EDTA | 0.10 | 0.1 |
| KATHON CG | 0.02 | 0.00033 |
| calcium chloride | 0.35 | 0.28 |
| perfume (proprietary composition) | 0.20 | 0.20 |
| grapefruit extracts* | 0.10 | 0.10* |
| Vitamin E acetate* | 0.20 | 0.20* |
| colorant 1 (proprietary composition) | 0.00006 | 0.00006 |
| colorant 2 (proprietary composition) | 0.0004 | 0.0004 |

(continued)

| Example Formulation 3B | | |
|---|---|---|
| | %W/W | %W/W |
| **Constituent** | **"as supplied"** | **active weight basis** |
| CIBAFAST H Liquid | 0.30 | 0.09 |
| TINOGARD Q | 0.30 | 0.03 |
| **Total** | **100.00** | **100.00** |
| pH = 3.70 | | |
| Viscosity = 4572 cPs | | |
| * = indicates a preparation "as supplied" from the respective supplier, actual active weight of identified constituent not specified by supplier | | |

The foregoing compositions may have included an additional minor amount of citric acid in order to adjust the pH of each composition to a pH in the range of 3.7 to 4.0. Each of the foregoing compositions exhibited a viscosity of between 1500 - 4700 cPs, measured using a Brookfield viscometer, LV spindle #3, at 12 rpm at room temperature (approx. 20-22°C). The foregoing compositions provide antimicrobial efficacy when topically applied and with the inclusion of both Vitamin E provides a skin nourishing effect and a conjunctive aromatherapy benefit from the presence of the grapefruit extracts.

| Example Formulation 4 | | |
|---|---|---|
| | % W/W | %W/W |
| **Constituent** | **"as supplied"** | **active weight basis** |
| deionized water | q.s. | q.s. |
| STEPANOL D-HS | 24.00 | 8.88 |
| salicylic acid | 0.10 | 0.1 |
| glycerin (USP grade) | 2.00 | 2.0 |
| Polyquaternium 7 | 0.20 | 0.2 |
| tetrasodium EDTA | 0.10 | 0.10 |
| KATHON CG | 0.02 | 0.00033 |
| calcium chloride | 0.50 | 0.40 |
| perfume (proprietary composition) | 0.20 | 0.20 |
| lavender extract* | 0.10 | 0.10* |
| grape extract (aqueous)* | 0.10 | 0.10* |
| colorant 1 | 0.00035 | 0.0005 |
| colorant 2 | 0.00015 | 0.0003 |
| **Total** | **100.00** | **100.00** |
| * = indicates a preparation "as supplied" from the respective supplier, actual active weight of identified constituent not specified by supplier | | |

| Example Formulation 4A | | |
|---|---|---|
| | %W/W | %W/W |
| **Constituent** | **"as supplied"** | **active weight basis** |
| deionized water | q.s. | q.s. |
| STEPANOL D-HS | 24.00 | 8.88 |
| salicylic acid | 0.10 | 0.1 |
| glycerin (USP grade) | 2.00 | 2.0 |
| Polyquaternium 7 | 0.20 | 0.2 |
| tetrasodium EDTA | 0.10 | 0.10 |
| KATHON CG | 0.02 | 0.00033 |
| calcium chloride | 0.75 | 0.60 |
| perfume (proprietary composition) | 0.20 | 0.20 |
| lavender extract* | 0.10 | 0.10* |
| grape extract (aqueous)* | 0.10 | 0.10* |
| colorant 1 | 0.00013 | 0.00013 |
| CIBAFAST H Liquid | 0.30 | 0.09 |
| TINOGARD Q | 0.30 | 0.03 |
| **Total** | **100.00** | **100.00** |
| * = indicates a preparation "as supplied" from the respective supplier, actual active weight of identified constituent not specified by supplier | | |

| Example Formulation 4B | | |
|---|---|---|
| | %W/W | %W/W |
| **Constituent** | **"as supplied"** | **active weight basis** |
| deionized water | q.s. | q.s. |
| STANDAPOL EA2 | 27.00 | 6.75 |
| VELVETEX BK-35 | 7.5 | 2.25 |
| salicylic acid | 0.10 | 0.1 |
| glycerin (USP grade) | 2.00 | 2.0 |
| Polyquaternium 7 | 0.20 | 0.2 |
| tetrasodium EDTA | 0.10 | 0.10 |
| KATHON CG | 0.02 | 0.00033 |
| calcium chloride | 0.35 | 0.28 |
| perfume (proprietary composition) | 0.20 | 0.20 |
| lavender extract* | 0.10 | 0.10* |
| grape extract (aqueous)* | 0.10 | 0.10* |
| colorant 1 | 0.00013 | 0.00013 |
| CIBAFAST H Liquid | 0.30 | 0.09 |
| TINOGARD Q | 0.30 | 0.03 |

(continued)

| Example Formulation 4B | | |
|---|---|---|
| | %W/W | %W/W |
| **Constituent** | **"as supplied"** | **active weight basis** |
| **Total** | **100.00** | **100.00** |
| pH = 3.70 | | |
| Viscosity = 5333 cPs | | |
| * = indicates a preparation "as supplied" from the respective supplier, actual active weight of identified constituent not specified by supplier | | |

The foregoing compositions may have included an additional minor amount of citric acid in order to adjust the pH of each composition to a pH in the range of 3.7 to 4.0. Each of the foregoing compositions exhibited a viscosity of between 1500 - 4500 cPs, measured using a Brookfield viscometer, LV spindle #3, at 12 rpm at room temperature (approx. 20-22°C). The foregoing compositions provide antimicrobial efficacy when topically applied and with the inclusion of both grape extract (*vitis vinifera*) provides an antioxidant effect and a conjunctive aromatherapy benefit from the presence of the lavender extract.

| Example Formulation 5 | | |
|---|---|---|
| | % W/W | %W/W |
| **Constituent** | **"as supplied"** | **active weight basis** |
| deionised water | q.s. | q.s. |
| STEPANOL D-HS | 24.0 | 8.88 |
| salicylic acid | 0.10 | 0.1 |
| glycerin (USP grade) | 2.00 | 2.0 |
| polyquaternium 7 | 0.20 | 0.2 |
| tetrasodium EDTA | 0.10 | 0.1 |
| KATHON CG | 0.02 | 0.00033 |
| ACUSOL OP301 | 0.20 | 0.076 |
| calcium chloride | 0.50 | 0.40 |
| perfume (proprietary composition) | 0.20 | 0.22 |
| cotton milk extract* | 0.10 | 0.1* |
| chamomile extract* | 0.10 | 0.1* |
| colorant 1 (proprietary composition) | 0.04 | 0.04 |
| colorant 2 (proprietary composition) | 0.02 | 0.02 |
| **Total** | **100.00** | **100.00** |
| * = indicates a preparation "as supplied" from the respective supplier, actual active weight of identified constituent not specified by supplier | | |

The foregoing composition was adjusted to a pH of 4.32 with a minor amount of an aqueous, 20% solution of citric acid composition which was pipetted into the stirring compositions as it was being prepared.

| Example Formulation 6 | | |
|---|---|---|
| | % W/W | %W/W |
| **Constituent** | **"as supplied"** | **active weight basis** |
| deionised water | q.s. | q.s. |
| STEPANOL D-HS | 24.0 | 8.88 |
| salicylic acid | 0.20 | 0.2 |
| glycerin (USP grade) | 2.00 | 2.0 |
| polyquaternium 7 | 0.20 | 0.2 |
| tetrasodium EDTA | 0.10 | 0.1 |
| KATHON CG | 0.02 | 0.00033 |
| calcium chloride | 0.50 | 0.40 |
| **Total** | **100.00** | **100.00** |

The foregoing composition was adjusted to a pH of 4.17 with a minor amount of an aqueous, 20% solution of citric acid composition which was pipetted into the stirring compositions as it was being prepared.

| Example Formulation 7 | | |
|---|---|---|
| | %W/W | %W/W |
| **Constituent** | **"as supplied"** | **active weight basis** |
| deionised water | q.s. | q.s. |
| STEPANOL D-HS | 24.0 | 8.88 |
| salicylic acid | 0.20 | 0.2 |
| glycerin (USP grade) | 2.00 | 2.0 |
| Polyquaternium 7 | 0.20 | 0.2 |
| tetrasodium EDTA (anhydrous powder) | 0.10 | 0.1 |
| KATHON CG | 0.02 | 0.00033 |
| ACUSOL OP301 | 0.20 | 0.076 |
| calcium chloride | 0.50 | 0.40 |
| perfume (proprietary composition) | 0.20 | 0.22 |
| cotton milk extract* | 0.10 | 0.1* |
| chamomile extract* | 0.10 | 0.1* |
| colorant 1 (proprietary composition) | 0.04 | 0.04 |
| colorant 2 (proprietary composition) | 0.02 | 0.02 |
| **Total** | **100.00** | **100.00** |
| * = indicates a preparation "as supplied" from the respective supplier, actual active weight of identified constituent not specified by supplier | | |

The foregoing composition was adjusted to a pH of 4.40 with a minor amount of an aqueous, 20% solution of citric acid composition which was pipetted into the stirring compositions as it was being prepared.

| Example Formulation 8 | | |
|---|---|---|
| | **%W/W** | **%W/W** |
| **Constituent** | **"as supplied"** | **active weight basis** |
| deionized water | q.s. | q.s. |
| STANDAPOL EA2 | 27.00 | 6.75 |
| VELVETEX BK-35 | 7.5 | 2.25 |
| salicylic acid | 0.10 | 0.1 |
| glycerin (USP grade) | 2.00 | 2.0 |
| Polyquaternium 7 | 0.20 | 0.2 |
| tetrasodium EDTA | 0.10 | 0.1 |
| KATHON CG | 0.02 | 0.00033 |
| calcium chloride | 0.06 | 0.60 |
| perfume (proprietary composition) | 0.22 | 0.20 |
| grapefruit extracts* | 0.10 | 0.10* |
| Vitamin E acetate* | 0.20 | 0.20* |
| colorant 1 (proprietary composition) | 0.00006 | 0.00006 |
| colorant 2 (proprietary composition) | 0.0004 | 0.0004 |
| CIBAFAST H Liquid | 0.30 | 0.09 |
| TINOGARD Q | 0.30 | 0.03 |
| **Total** | **100.00** | **100.00** |
| pH = 3.66 | | |
| * = indicates a preparation "as supplied" from the respective supplier, actual active weight of identified constituent not specified by supplier | | |

The foregoing composition was adjusted to a pH of 3.66 with a minor amount of an aqueous, 20% solution of citric acid composition which was pipetted into the stirring compositions as it was being prepared.

| Example Formulation 9 | | |
|---|---|---|
| | **%W/W** | **%W/W** |
| **Constituent** | **"as supplied"** | **active weight basis** |
| deionized water | q.s. | q.s. |
| STANDAPOL EA2 | 27.00 | 6.75 |
| VELVETEX BK-35 | 7.50 | 2.25 |
| salicylic acid | 0.10 | 0.1 |
| glycerin (USP grade) | 2.00 | 2.0 |
| Polyquaternium 7 | 0.20 | 0.2 |
| tetrasodium EDTA | 0.10 | 0.1 |
| KATHON CG | 0.02 | 0.00033 |
| calcium chloride | 0.06 | 0.28 |
| perfume (proprietary composition) | 0.20 | 0.20 |
| grapefruit extracts* | 0.10 | 0.10* |

(continued)

| Example Formulation 9 | | |
|---|---|---|
| | %W/W | %W/W |
| Constituent | "as supplied" | active weight basis |
| Vitamin E acetate* | 0.20 | 0.20* |
| colorant 1 (proprietary composition) | 0.00006 | 0.00006 |
| colorant 2 (proprietary composition) | 0.0004 | 0.0004 |
| CIBAFAST H Liquid | 0.30 | 0.09 |
| TINOGARD Q | 0.30 | 0.03 |
| **Total** | **100.00** | **100.00** |
| pH = 3.75 | | |
| * = indicates a preparation "as supplied" from the respective supplier, actual active weight of identified constituent not specified by supplier | | |

The foregoing composition was adjusted to a pH of 3.66 with a minor amount of an aqueous, 20% solution of citric acid composition which was pipetted into the stirring compositions as it was being prepared.

The following three formulations were prepared as comparative example formulations which either included no acid constituent or which included lactic acid in place of salicylic acid which was absent from both of the following compositions.

| Comparative Formulation C1 | | |
|---|---|---|
| Constituent | %W/W | %W/W |
| | "as supplied" | active weight basis |
| deionised water | q.s. | q.s. |
| STEPANOL D-HS | 24.00 | 8.88 |
| lactic acid (90%wt actives) | 0.25 | 0.22 |
| glycerin (USP grade) | 2.00 | 2.0 |
| Polyquaternium 7 | 0.20 | 0.2 |
| tetrasodium EDTA | 0.10 | 0.1 |
| KATHON CG | 0.02 | 0.2 |
| ACUSOL OP301 | 0.20 | 0.076 |
| calcium chloride | 0.50 | 0.4 |
| **Total** | **100.00** | **100.00** |
| * = indicates a preparation "as supplied" from the respective supplier, actual active weight of identified constituent not specified by supplier | | |

The foregoing composition of C1 was adjusted to a pH of 4.56 with a minor amount of an aqueous, 20% solution of citric acid composition which was pipetted into the stirring compositions as it was being prepared.

| Comparative Formulation C2 | | |
|---|---|---|
| Constituent | %W/W | %W/W |
| | "as supplied" | active weight basis |
| deionised water | q.s. | q.s. |

(continued)

| Comparative Formulation C2 | | |
|---|---|---|
| **Constituent** | **%W/W** | **%W/W** |
| | **"as supplied"** | **active weight basis** |
| STEPANOL D-HS | 24.00 | 8.88 |
| salicylic acid | --- | --- |
| glycerin (USP grade) | 2.00 | 2.0 |
| Polyquaternium 7 | 0.20 | 0.20 |
| tetrasodium EDTA | 0.10 | 0.10 |
| KATHON CG | 0.02 | 0.04 |
| calcium chloride | 0.50 | 0.40 |
| **Total** | **100.00** | **100.00** |
| * = indicates a preparation "as supplied" from the respective supplier, actual active weight of identified constituent not specified by supplier | | |

The foregoing composition of C2 was adjusted to a pH of 4.58 with a minor amount of an aqueous, 20% solution of citric acid composition which was pipetted into the stirring compositions as it was being prepared.

| Comparative Formulation C3 | | |
|---|---|---|
| | **%W/W** | **%W/W** |
| **Constituent** | **"as supplied"** | **active weight basis** |
| deionised water | q.s. | q.s. |
| STEPANOL D-HS | 24.0 | 8.88 |
| salicylic acid | 0.10 | 0.1 |
| glycerin (USP grade) | 2.00 | 2.0 |
| Polyquaternium 7 | 0.20 | 0.2 |
| tetrasodium EDTA | 0.10 | 0.1 |
| KATHON CG | 0.02 | 0.00033 |
| ACUSOL OP301 | 0.20 | 0.076 |
| calcium chloride | 0.50 | 0.40 |
| perfume (proprietary composition) | 0.20 | 0.22 |
| cotton milk extract* | 0.10 | 0.1* |
| chamomile extract* | 0.10 | 0.1* |
| colorant 1 | 0.04 | 0.04 |
| colorant 2 | 0.02 | 0.02 |
| **Total** | **100.00** | **100.00** |
| * = indicates a preparation "as supplied" from the respective supplier, actual active weight of identified constituent not specified by supplier | | |

The foregoing composition was adjusted to a pH of 5.66 with a minor amount of an aqueous, 20% solution of citric acid composition which was pipetted into the stirring compositions as it was being prepared.

Antimicrobial Efficacy:

Certain of the compositions indicated above were evaluated for their antimicrobial efficacy against various microorganism selected from: *Staphylococcus aureus* (ATCC 6538), *Escherichia coli* (ATCC 10536), *Salmonella choleraesuis* (ATCC 13311), *Campylobacter jejuni* (ATCC 29428) and *Candida albicans* (ATCC 10231) in accordance with the following general protocol which based in part on the "Standard Test for the Assessment of the Rapid Germicidal Activity for Antibacterial Liquid and Bar Soap Products" published by the Soap and Detergent Association (USA) 1995 draft, an on "prEN12054 - Chemical Disinfection and Antiseptics - Products for Hygienic and Surgical Handrub and Handwash, Bactericidal Activity - Test Methods and Requirements (Phase 2, Step 1)".

The specific protocol of the test, as well as materials utilized are set forth in the following detailed description. While no established criteria is considered to be a standard for a "pass" or "fail" determination, it is considered by the inventors that any tested formulation which fails to provide a $\log_{10}$ reduction of less than 1 is considered to be a "fail" score, while any formulation which provided a $\log_{10}$ reduction of 3 or more is considered to be "superior" performing formulations. Formulations which provide a $\log_{10}$ reduction of at least 1 are considered to be a "pass" score, although are less preferable to formulations providing a $\log_{10}$ reduction of 3 or more.

Testing and Evaluation Protocol

Preparation of Test Medium:

A tryptic sodium chloride solution was produced by providing 1g of tryptone, pancreatic digest of casein, and 8.5 g of sodium chloride to 1 liter of deionized water.

Tryptic soy agar dispensed as ~400ml aliquots.

Neutralization liquid can be any validated mixture such as D/E Broth.

Hard water for product dilution (300ppm $CaC0_3$)

A "Solution A" was prepared by dissolving 19.84 g of anhydrous $MgCl_2$ and 46.24g of anhydrous $CaCl_2$ in purified water, which was then dilute to 1 liter in a volumetric flask.

A "Solution B" was prepared by dissolving 35.02 g of $NaHCO_3$ in purified water and dilute to 1 liter in a volumetric flask.

To 600 ml of sterile purified water was added 6 ml of solution A, and 8 ml of solution B, which was then diluted to 1 liter with purified water using a volumetric flask. The final solution was sterilized by passing through a membrane filter with an effective pore size of 0.45μm or 0.2μm. The pH of this solution was between 6.8 and 7.2 at 25°C. Where necessary, the hard water was adjusted using 1N HCL or 1N NaOH.

The prepared Solutions A and B may be stored at 2°C to 8°C for up to one month.

Test Organisms

The test organisms used were from the group which included: *Staphylococcus aureus* (ATCC 6538), *Escherichia coli* (ATCC 10536), *Salmonella choleraesuis* (ATCC 13311), *Campylobacter jejuni* (ATCC 29428) and *Candida albicans* (ATCC 10231).

The working cultures used were either a second or third generation subculture on TSA slopes from frozen beads. Subcultures were prepared on TSA slopes and incubated at 35±2.5°C for 18-24 hours.

Preparation of Test Cultures

At least 2 loopfuls of the working slant were removed and the cells were suspended in approximately 10ml of TSC and rotated at a revolution rate of 150 $min^{-1}$ for at least three minutes.

A portion of the suspended cells were pipetted and added to an appropriate volume of TSC (usually 9ml or more depending on the amount of samples tested). Adjustments were made in order to provide 1.5 -5.0$x10^8$cfu $ml^{-1}$ for each organism using for example the biolog transmittance kit. A typical absorbance range between 45 and 50 (biolog) was used for each organism.

These were the test cultures adjusted to the correct level for each organism.

Preparation of Sample

All samples were tested at a respective dilution of 50%v/v of the product sample in hard water. As an example, a final testing solution of 50%v/v was achieved when the 1 ml of test culture is added, i.e. 5ml sample, 4ml hard water and 1ml test culture.

Temperature

Test solutions were maintained at a controlled temperature of 37 ±1°C in a shaking waterbath unless otherwise stated.

Testing Procedure

The following steps were performed at 37°C±1°C.

1. In a 20ml McCartney bottle was added 4ml hard water to 5ml of sample
2. The sample mixture was allowed to reach thermal equilibrium in a waterbath. Also provided to the waterbath maintained at 37°C±1°C was a tube of 8ml D/E broth and 1ml sterile deionized water for each sample mixture tested.
3. Thereafter 1ml of test culture was added to each sample mixture and vortexed for 5 seconds, which were thereafter left for 1 minute ± 10 second contact time.
4. At the conclusion of the contact time the 1ml of the test mixture was removed to to 9ml of neutralizer, the resultant inactivated mixture formed a $10^{-1}$ dilution of the test reaction mixture(1ml in 9ml is the standard neutralization dilution. Where this is not applicable due to insufficient neutralization a 0.1ml in 9.9ml neutralization may be used).
5. A 5 minute neutralization period was allowed, and thereafter serial dilutions of 1 in 9ml TSC were performed in order to enumerate the surviving organisms. Plates were poured with molten TSA.
6. Steps 1 to 5 were repeated using a maximum of 3 samples per 1 minute contact time run and thus a 20 second interval between each.
7. The plated were incubate at 35±2.5°C for 2-3 days and thereafter the colonies were counted.
8. Optionally the test was repeated on at least two occasions, preferably three occasions using a different inoculum for each.

Counting of Test Cultures

The test cultures were counted as follows.

1. Each organism was diluted to $10^{-7}$ in TSC using appropriate dilutions.
2. Thereafter the $10^{-6}$ and $10^{-7}$ dilutions were plated in duplicate and TSA was poured on.
3. The samples (replicates) were incubated at 35±2.5°C for 2-3 days and thereafter the colonies were counted.

Validation of Neutralization

The following was conducted for each organism/test substance combination.

1. Neutralization wasvalidated at least once for each new formulation/organism tested.
2. Prior to the test procedure the test culture was diluted using TSC to give a cell concentration of $10^3$ cfu/ml by performing five ten-fold dilutions in TSC.
3. The test solutions (5ml sample, 4ml hard water) and 8ml D/E broth tube were allowed to thermally qquilibrate in the waterbath for 5 minutes.
4. 1ml of TSC was added to each sample solution, vortexed and left for 1 minute.
5. 1ml of the test mixture was removed to 8ml neutralizer, vortexed and left for 5 minutes.
6. 1ml of the test culture (as per step 2) was added to the neutralized mixture and leave for 5 minutes.
7. The neutralized mixture was diluted using serial dilutions in TSC and plates poured in TSA.
8. Steps 3 to 7 were repeated but, replacing product sample with hard water as a control.
9. The samples were incubated at 35±2.5°C for 2-3 days and thereafter colonies counted.

Neutralization was considered to be effective if the number of organisms in the inactivated product mixture was at least ±0.5 log of that recovered from the control.

Calculation and Reporting of Test Results

Where possible plates containing between 15 and 300 cfu $ml^{-1}$ were used to determine colony counts.
The weighted mean is the average of choice, and is used when two dilutions have colonies in the range 15 - 300 cfu $ml^{-1}$.

Calculation of microbiocidal effect (log reduction)

The microbiocidal effect (ME) due to the action of the product over the test contact time at the temperature at which the test was performed is expressed by the formula:

$$ME = \text{Log } Nc - \text{Log } Nd$$

where:

Nc = Number of cfu/ml of the test culture count.
Nd = Number of cfu/ml of the sample count.

The ME was calculated to 2 decimal places and reported.

The composition according to Example Formulation 2, at a pH of 4.37, as disclosed above was evaluated for antimicrobial efficacy against each of *Staphylococcus aureus* (ATCC 6538), *Escherichia coli* (ATCC 10536), *Salmonella choleraesuis* (ATCC 13311), *Campylobacterjejuni* (ATCC 29428) and *Candida albicans* (ATCC 10231) in accordance with the foregoing testing protocol. Its efficacy was determined, and the results are reported on the following table.

| Antimicrobial Efficacy - I | |
|---|---|
| Example Formulation 2, pH = 4.37 | |
| (mean) $\log_{10}$ reduction against *S.aureus* | >4.41 |
| (mean) $\log_{10}$ reduction against *E.coli* | >4.54 |
| (mean) $\log_{10}$ reduction against *S.choleraesuis* | 3.62 |
| (mean) $\log_{10}$ reduction against *C.jejuni* | >2.40 |
| (mean) $\log_{10}$ reduction against *C.albicans* | 2.44 |

As is evident from the foregoing, the tested composition provided greater than 3 $\log_{10}$ reduction (>99.9%) against *Staphylococcus aureus* (ATCC 6538), *Escherichia coli* (ATCC 10536), *Salmonella choleraesuis* (ATCC 13311), while providing a reasonable degree, viz. at least 2 $\log_{10}$ reduction against *Campylobacter jejuni* (ATCC 29428) and *Candida albicans* (ATCC 10231).

Further of the compositions disclosed above were evaluated for their antimicrobial efficacy against both *Staphylococcus aureus* (ATCC 6538) and *Escherichia coli* (ATCC 10536) in accordance with the foregoing testing protocol and the their efficacy was determined, and are reported on the following table.

| Antimicrobial Efficacy - II | | | |
|---|---|---|---|
| | | | |
| Formulation | pH of formulation | (mean) $\log_{10}$ reduction against *S.aureus* | (mean) $\log_{10}$ reduction against *E.coli* |
| Ex.3B | 3.70 | > 4.22 | > 4.28 |
| Ex.5 | 4.32 | > 3.97 | 3.75 |
| Ex.6 | 4.17 | > 3.97 | 4.54 |
| Ex.7 | 4.40 | > 3.97 | 4.54 |
| Ex.8 | 3.66 | > 4.30 | > 4.80 |
| Ex.9 | 3.75 | > 4.22 | > 4.28 |
| C1 | 4.56 | 3.26 | 0.84 |
| C2 | 4.58 | 3.38 | 0.28 |

(continued)

| Formulation | pH of formulation | (mean) $log_{10}$ reduction against *S.aureus* | (mean) $log_{10}$ reduction against *E.coli* |
|---|---|---|---|
| C3 | 5.66 | 0.84 | 0.50 |

As is evident from the results reported above, the formulations (Ex.3B, Ex. 5 - Ex. 9) which are demonstrative of the invention and are among the preferred embodiments provided reduction of both gram positive and gram negative microorganisms and were superior in performance. The comparative formulation, C1 which included lactic acid but no salicylic acid demonstrated excellent results against *Staphylococcus aureus* but failed against *Escherichia coli*. Comparative formulation, C2 which included neither salicylic acid or lactic acid also demonstrated excellent results against *Staphylococcus aureus* but failed against *Escherichia coli*. Comparative formulation C3, having a higher pH, failed against both *Staphylococcus aureus* and *Escherichia coli*.

Virucidal Efficacy:

The composition according to Example Formulation 2, at a pH of 4.37, as disclosed above was evaluated for antimicrobial efficacy against a strain of the influenza virus, Influenza A (H1N1), an avian orthomyxovirus (ATCC VR-98) in accordance with the EPA Virucidal Test Method - "Liquid and Spray Disinfectants against Hemaglutinnating Viruses Which are Assayed in Embryonated Chick Eggs".
As a result of the test it was demonstrated that the composition according to the Example Formulation 2 at a temperature of 37°C demonstrated complete inactivation of the Influenza A (H1N1) virus with a greater than 5 $log_{10}$ reduction (>99.999%) at 1 minute contact time.
The composition according to Example Formulation 2, at a pH of 4.37, as disclosed above was evaluated for antimicrobial efficacy against a strain of the Human Rhinovirus (Picornavirus), Rhinovirus Type 39 (ATCC VR-340) in accordance with the EPA Virucidal Test Method - "Liquid and Spray Disinfectants against Viruses which Require Standard Assay Conditions".
As a result of the test it was demonstrated that the composition according to the Example Formulation 2 demonstrated a 2.91 $log_{10}$ reduction (>99.88%) of the Rhinovirus at 1 minute contact time.

**Claims**

**1.** A topical composition for the application to the human body having a viscosity of at least 500 cps and a pH of from about 3 to less than 7, which comprises:

one or more anionic surfactants;
optionally one or more alkanolamides;
optionally one or more further surfactants selected from nonionic and amphoteric surfactants;
one or more emollients;
salicylic acid and/or salicylate in a sufficient amount such that the compositions are effective against both gram positive and gram negative microorganisms;
one or more water-soluble inorganic or organic salts, and
water.

**2.** A topical composition according to claim 1 wherein the anionic surfactant is selected from: alkyl sulfates, alpha-olefin sulfonates, alkyl ether sulfate, and mixtures thereof.

**3.** A topical composition according to any preceding claim wherein the anionic surfactant constituent comprises a $C_{12}$-$C_{16}$ olefin sulfonates or salt form thereof.

**4.** A topical composition according to any preceding claim wherein the anionic surfactant constituent comprises a $C_{12}$-$C_{16}$ ether sulfate or salt form thereof.

**5.** A topical composition according to any preceding claim wherein the anionic surfactant constituent comprises an ammonium lauryl sulfate, sodium lauryl ether sulfate, and lauramide DEA

**6.** A topical composition according to any preceding claim wherein the one or more anionic surfactants are present in

amounts of from 0.1 - 15 %wt.

**7.** A topical composition according to any preceding claim which includes a nonionic surfactant.

**8.** A topical composition according to any preceding claim wherein the emollient comprises one or more emollients selected from cationic Polyquaternium-type polymers and alkyl polyhydroxy compounds.

**9.** A topical composition according to any preceding claim which comprises from 0.05 - 5%wt of emollients.

**10.** A topical composition according to any preceding claim wherein the salicylic acid and/or salicylates is present in at least three, preferably at least four times the amount of the total mass of other organic acid or inorganic acid constituents which may be present in the topical compositions.

**11.** A topical composition according to any of claims 1 - 9 wherein the salicylic acid and/or salicylate are present in the absence of other organic acid or inorganic acid constituents.

**12.** A topical composition according to any preceding claim wherein the one or more water-soluble inorganic or organic salts are one or more inorganic chloride salts.

**13.** A topical composition according to any preceding claim wherein inorganic sodium salts are excluded.

**14.** A topical composition according to any preceding claim which further comprises one or more chelating agents.

**15.** A topical composition according to any preceding claim which further comprises one or more latexes.

**16.** A topical composition according to any preceding claim which further comprises one or more preservatives.

**17.** A topical composition according to any preceding claim which further comprises one or more fragrances.

**18.** A topical composition according to any preceding claim which further comprises one or more colorants.

**19.** A topical composition according to any preceding claim which further comprises one or more humectants.

**20.** A topical composition according to any preceding claim which further comprises one or more essential oils.

**21.** A topical composition according to any preceding claim which further comprises one or more antioxidants.

**22.** A topical composition according to any preceding claim which further comprises one or more milk protein compounds and/or a milk protein hydrolysates.

**23.** A topical composition according to any preceding claim which further comprises cotton milk.

**24.** A topical composition according to any preceding claim which further comprises aloe vera.

**25.** A topical composition according to any preceding claim which exhibits a viscosity of 1000 to about 10,000 cps.

**26.** A topical composition according to claim 25 which exhibits a viscosity of from about 1500 to 7000 cps.

**27.** A topical composition according to any preceding claim which exhibits antimicrobial efficacy against both *Staphylococcus aureus* and *Escherichia coli.*

**28.** A topical composition according to claim 27 which exhibits antimicrobial on the order of at least a 2 $log_{10}$ reduction against both *Staphylococcus aureus* and *Escherichia coli*.

**29.** A topical composition according to claim 28 which exhibits antimicrobial on the order of at least a 3 $log_{10}$ reduction against both *Staphylococcus aureus* and *Escherichia coli*.

**30.** A topical composition according to claim 25 which exhibits antiviral efficacy.

31. A topical composition according to any preceding claim which has a pH of from about 3 to 6.

**Patentansprüche**

1. Topische Zusammensetzung zur Anwendung fiir den menschlichen Körper mit einer Viskosität von mindestens 500 cps und einem pH von etwa 3 bis weniger als 7, die Folgendes umfasst:

   ein oder mehrere anionische(s) oberflächenaktive(s) Mittel;
   wahlweise ein oder mehrere Alkanolamid(e);
   wahlweise ein oder mehrere weitere(s) oberflächenaktive(s) Mittel, ausgewählt aus nicht-ionischen und amphoteren oberflächenaktiven Mitteln;
   ein oder mehrere Weichmacher;
   Salicylsäure und/oder Salicylat in einer derart ausreichenden Menge, dass die Zusammensetzungen sowohl gegen grampositive als auch gegen gramnegative Mikroorganismen wirksam sind;
   ein oder mehrere wasserlösliche(s) anorganische(s) oder organische(s) Salz(e) und Wasser.

2. Topische Zusammensetzung nach Anspruch 1, wobei das anionische oberflächenaktive Mittel ausgewählt ist aus: Alkylsulfaten, alpha-Olefinsulfonaten, Alkylethersulfat und Gemischen davon.

3. Topische Zusammensetzung nach einem der vorangehenden Ansprüche, wobei der anionische oberflächenaktive Bestandteil ein $C_{12}$-$C_{16}$-Olefinsulfonat oder Salz davon umfasst.

4. Topische Zusammensetzung nach einem der vorangehenden Ansprüche, wobei der anionische oberflächenaktive Bestandteil ein $C_{12}$-$C_{16}$-Ethersulfat oder Salz davon umfasst.

5. Topische Zusammensetzung nach einem der vorangehenden Ansprüche, wobei der anionische oberflächenaktive Bestandteil ein Ammoniumlaurylsulfat, Natriumlaurylethersulfat und Lauramid-DEA umfasst.

6. Topische Zusammensetzung nach einem der vorangehenden Ansprüche, wobei das eine oder die mehreren anionische(n) oberflächenaktive(n) Mittel in Mengen von 0,1 bis 15 Gew.-% vorliegt bzw. vorliegen.

7. Topische Zusammensetzung nach einem der vorangehenden Ansprüche, die ein nicht-ionisches oberflächenaktives Mittel einschließt.

8. Topische Zusammensetzung nach einem der vorangehenden Ansprüche, wobei der Weichmacher einen oder mehrere Weichmacher, ausgewählt aus kationischen Polymeren vom Polyquatemiumtyp und Alkylpolyhydroxyverbindungen, umfasst.

9. Topische Zusammensetzung nach einem der vorangehenden Ansprüche, die 0,05-5 Gew.-% Weichmacher umfasst.

10. Topische Zusammensetzung nach einem der vorangehenden Ansprüche, wobei die Salicylsäure und/oder die Salicylate in einer mindestens dreifachen, vorzugsweise mindestens vierfachen Menge der Menge der Gesamtmasse von anderen organischen Säure- und anorganischen Säurebestandteilen, die in den topischen Zusammensetzungen vorliegen können, vorliegen.

11. Topische Zusammensetzung nach einem der Ansprüche 1-9, wobei die Salicylsäure und/oder das Salicylat in der Abwesenheit von anderen organischen oder anorganischen Säurebestandteilen vorliegen.

12. Topische Zusammensetzung nach einem der vorangehenden Ansprüche, wobei es sich bei dem einen oder den mehreren wasserlöslichen anorganischen oder organischen Salz(en) um ein oder mehrere anorganische(s) Chloridsalz(e) handelt.

13. Topische Zusammensetzung nach einem der vorangehenden Ansprüche, wobei anorganische Natriumsalze ausgeschlossen sind.

14. Topische Zusammensetzung nach einem der vorangehenden Ansprüche, die ferner einen oder mehrere Chelatbildner umfasst.

**15.** Topische Zusammensetzung nach einem der vorangehenden Ansprüche, die ferner einen oder mehrere Latex bzw. Latizes umfasst.

**16.** Topische Zusammensetzung nach einem der vorangehenden Ansprüche, die ferner ein oder mehrere Konservierungsmittel umfasst.

**17.** Topische Zusammensetzung nach einem der vorangehenden Ansprüche, die ferner einen oder mehrere Duftstoff (e) umfasst.

**18.** Topische Zusammensetzung nach einem der vorangehenden Ansprüche, die ferner ein oder mehrere Farbmittel umfasst.

**19.** Topische Zusammensetzung nach einem der vorangehenden Ansprüche, die ferner ein oder mehrere Feuchtmittel umfasst.

**20.** Topische Zusammensetzung nach einem der vorangehenden Ansprüche, die ferner ein oder mehrere essentielle (s) Öl(e) umfasst.

**21.** Topische Zusammensetzung nach einem der vorangehenden Ansprüche, die ferner ein oder mehrere Antioxidationsmittel umfasst.

**22.** Topische Zusammensetzung nach einem der vorangehenden Ansprüche, die ferner eine oder mehrere Milchproteinverbindung(en) und/oder ein Milchproteinhydrolysat umfasst.

**23.** Topische Zusammensetzung nach einem der vorangehenden Ansprüche, die ferner Baumwollmilch umfasst.

**24.** Topische Zusammensetzung nach einem der vorangehenden Ansprüche, die ferner Aloe Vera umfasst.

**25.** Topische Zusammensetzung nach einem der vorangehenden Ansprüche, die eine Viskosität von 1000 bis etwa 10.000 cps aufweist.

**26.** Topische Zusammensetzung nach Anspruch 25, die eine Viskosität von etwa 1500 bis 7000 cps aufweist.

**27.** Topische Zusammensetzung nach einem der vorangehenden Ansprüche, die antimikrobielle Wirksamkeit sowohl gegen *Staphylococcus aureus* als auch gegen *Escherichia coli* aufweist.

**28.** Topische Zusammensetzung nach Anspruch 27, die antimikrobielle Wirksamkeit im Bereich einer Reduktion von mindestens 2 $\log_{10}$ sowohl gegen *Staphylococcus aureus* als auch gegen *Escherichia coli* aufweist.

**29.** Topische Zusammensetzung nach Anspruch 28, die antimikrobielle Wirksamkeit im Bereich einer Reduktion von mindestens 3 $\log_{10}$ sowohl gegen *Staphylococcus aureus* als auch gegen *Escherichia coli* aufweist.

**30.** Topische Zusammensetzung nach Anspruch 25, die antivirale Wirksamkeit aufweist.

**31.** Topische Zusammensetzung nach einem der vorangehenden Ansprüche, die einen pH von etwa 3 bis 6 aufweist.

## Revendications

**1.** Composition topique pour l'application au corps humain, ayant une viscosité d'au moins 500 cps et un pH d'environ 3 à moins de 7, qui comprend :

un ou plusieurs agents tensioactifs anioniques ;
facultativement un ou plusieurs alcanolamides ;
facultativement un ou plusieurs agents tensioactifs supplémentaires choisis entre des agents tensioactifs non ioniques et des agents tensioactifs amphotères ;
un ou plusieurs émollients ;
de l'acide salicylique et/ou un salicylate en une quantité suffisante de telle sorte que les compositions soient

efficaces à la fois contre des microorganismes Gram-positifs et des microorganismes Gram-négatifs ;
un ou plusieurs sels inorganiques ou organiques hydrosolubles ; et
de l'eau.

**2.** Composition topique suivant la revendication 1, dans laquelle l'agent tensioactif anionique est choisi entre : des alkylsulfates, des alpha-oléfine-sulfonates, un alkyléthersulfate et leurs mélanges.

**3.** Composition topique suivant l'une quelconque des revendications précédentes, dans laquelle le constituant tensioactif anionique comprend un oléfinesulfonate en $C_6$ à $C_{12}$ ou une forme de sel de celui-ci.

**4.** Composition topique suivant l'une quelconque des revendications précédentes, dans laquelle le constituant tensioactif anionique comprend un éthersulfate en $C_{12}$ à $C_{16}$ ou une forme de sel de celui-ci.

**5.** Composition topique suivant l'une quelconque des revendications précédentes, dans laquelle le constituant tensioactif anionique comprend un laurylsulfate d'ammonium, un lauryléthersulfate de sodium, et du lauramide-DEA.

**6.** Composition topique suivant l'une quelconque des revendications précédentes, dans laquelle le ou les agents tensioactifs anioniques sont présents en des quantités de 0,1 à 15 % en poids.

**7.** Composition topique suivant l'une quelconque des revendications précédentes, qui comprend un agent tensioactif non ionique.

**8.** Composition topique suivant l'une quelconque des revendications précédentes, dans laquelle l'émollient comprend un ou plusieurs émollients choisis entre des polymères du type Polyquaternium cationique et des composés alkylpolyhydroxyliques.

**9.** Composition topique suivant l'une quelconque des revendications précédentes, qui comprend 0,05 à 5 % en poids d'émollients.

**10.** Composition topique suivant l'une quelconque des revendications précédentes, dans laquelle l'acide salicylique et/ou les salicylates sont présents en au moins trois, de préférence au moins quatre, fois la quantité de la masse totale des autres constituants du type acide organique ou acide inorganique qui peuvent être présents dans les compositions topiques.

**11.** Composition topique suivant l'une quelconque des revendications 1 à 9, dans laquelle l'acide salicylique et/ou le salicylate sont présents en l'absence d'autres constituants du type acide organique ou acide inorganique.

**12.** Composition topique suivant l'une quelconque des revendications précédentes, dans laquelle le ou les sels inorganiques ou organiques hydrosolubles consistent en un ou plusieurs chlorures inorganiques.

**13.** Composition topique suivant l'une quelconque des revendications précédentes, dans laquelle des sels de sodium inorganiques sont exclus.

**14.** Composition topique suivant l'une quelconque des revendications précédentes, qui comprend en outre un ou plusieurs agents chélatants.

**15.** Composition topique suivant l'une quelconque des revendications précédentes, qui comprend en outre un ou plusieurs latex.

**16.** Composition topique suivant l'une quelconque des revendications précédentes, qui comprend en outre un ou plusieurs conservateurs.

**17.** Composition topique suivant l'une quelconque des revendications précédentes, qui comprend en outre un ou plusieurs parfums.

**18.** Composition topique suivant l'une quelconque des revendications précédentes, qui comprend en outre un ou plusieurs colorants.

**19.** Composition topique suivant l'une quelconque des revendications précédentes, qui comprend en outre un ou plusieurs agents humidifiants.

**20.** Composition topique suivant l'une quelconque des revendications précédentes, qui comprend en outre une ou plusieurs huiles essentielles.

**21.** Composition topique suivant l'une quelconque des revendications précédentes, qui comprend en outre un ou plusieurs antioxydants.

**22.** Composition topique suivant l'une quelconque des revendications précédentes, qui comprend en outre un ou plusieurs composés protéiques du lait et/ou hydrolysats de protéine du lait.

**23.** Composition topique suivant l'une quelconque des revendications précédentes, qui comprend en outre du lait de cotonnier.

**24.** Composition topique suivant l'une quelconque des revendications précédentes, qui comprend en outre de l'aloe vera.

**25.** Composition topique suivant l'une quelconque des revendications précédentes, qui a une viscosité de 1000 à environ 10 000 cps.

**26.** Composition topique suivant la revendication 25, qui présente une viscosité d'environ 1500 à 7000 cps.

**27.** Composition topique suivant l'une quelconque des revendications précédentes, qui présente une efficacité antimicrobienne à la fois contre *Staphylococcus aureus* et *Escherichia coli*.

**28.** Composition topique suivant la revendication 27, qui présente une efficacité antimicrobienne de l'ordre d'une réduction d'au moins 2 $\log_{10}$ contre à la fois *Staphylococcus aureus* et *Escherichia coli*.

**29.** Composition topique suivant la revendication 28, qui présente une efficacité antimicrobienne de l'ordre d'une réduction d'au moins 3 $\log_{10}$ contre à la fois *Staphylococcus aureus* et *Escherichia coli*.

**30.** Composition topique suivant la revendication 25, qui présente une efficacité antivirale.

**31.** Composition topique suivant l'une quelconque des revendications précédentes, qui a un pH d'environ 3 à 6.